(19) Europäisches Patentamt
European Patent Office
Office européen des brevets

(11) **EP 4 328 585 B1**

(12) # EUROPEAN PATENT SPECIFICATION

(45) Date of publication and mention
of the grant of the patent:
**28.01.2026 Bulletin 2026/05**

(21) Application number: **21937796.7**

(22) Date of filing: **19.04.2021**

(51) International Patent Classification (IPC):
**G01N 33/22** $^{(2006.01)}$

(52) Cooperative Patent Classification (CPC):
**G01N 33/225; G01N 21/45**

(86) International application number:
**PCT/JP2021/015847**

(87) International publication number:
**WO 2022/224297 (27.10.2022 Gazette 2022/43)**

(54) **CALCULATION DEVICE, CALCULATION METHOD, AND PROGRAM**

BERECHNUNGSVORRICHTUNG, BERECHNUNGSVERFAHREN UND PROGRAMM

DISPOSITIF DE CALCUL, PROCÉDÉ DE CALCUL ET PROGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.02.2024 Bulletin 2024/09**

(73) Proprietor: **Riken Keiki Co., Ltd.
Tokyo 174-8744 (JP)**

(72) Inventor: **ISHIGURO Tomoo
Kasukabe-shi, Saitama 344-0057 (JP)**

(74) Representative: **TBK
Bavariaring 4-6
80336 München (DE)**

(56) References cited:
**WO-A1-2016/104270** **WO-A1-2017/013897**
**WO-A1-2020/148927** **WO-A1-2020/148927**

JP-A- 2021 004 891     JP-B2- 6 402 387
JP-B2- 6 402 387

• **NAOKI NAKAMURA: "Development and
verification results of optsonic calorimeter to
solve field problems", INSTRUMENTATION
CONTROL ENGINEERING, KOGYO GIJUTSU-
SHA, TOKYO, JP, vol. 57, no. 11, 1 November
2014 (2014-11-01), JP**
, pages 21 - 24, XP009522570, ISSN: 0368-5780
• **MATSUSHITA TAKURO; KOICHIRO TSUGANE;
TOMOO ISHIGURO: "Genba Needs o Tsuikyu
shita Field Keiso Tool -Kaihatsu no Nerai to
Un'yo [Kaihatsu·Donyu Jirei] Zatsu Gas Seibun
no Eikyo o Jokyo suru Netsuryo Sokutei Gijutsu
[Calorimetry technology that removes the effects
of miscellaneous gas components]",
INSTRUMENTATION CONTROL ENGINEERING,
KOGYO GIJUTSU-SHA, TOKYO, JP, vol. 53, no. 5,
1 May 2010 (2010-05-01), JP**
, pages 64 - 66, XP009503884, ISSN: 0368-5780

**Description**

Technical Field

The present invention relates to a calculation device, a computer-implemented calculation method, and a program that can calculate various values relating to combustion of a target gas (for example, a natural gas).

Background Art

For the handling of combustible gases, there are many physical property values that serve as an index or a standard, and various calculation methods are used for each physical property value.

One of the physical property values of combustible gases is a heating value. Among the methods of measuring the heating value, there has been known a method in which the heating value of a target gas, which is the gas subjected to heating value measurement, is calculated on the basis of a refractive index-converted heating value that is obtained from the refractive index of the target gas and on a sound speed converted-heating value obtained from the sound speed of the target gas (see, for example, PTL 1).

Examples of other physical property values include a compression factor and a combustion speed. To calculate these values, methods that conform to international standards are generally used. In this case, for example, composition analysis values of gas chromatography are generally used as input values (parameters).

For calculating the compression factor of a natural gas, methods according to ISO12213-2 and ISO12213-3 are widely known, for example.

The calculation method according to ISO12213-2 is a method of calculating a compression factor Z using a pressure P and a temperature T of a natural gas, and a molar concentration $x_i$ of each component that composes the natural gas as input values (input parameters).

The calculation method according to ISO12213-3 uses a pressure P and a temperature T of a natural gas, a heating value Hs (a gross heating value converted to a measurement reference state temperature of 0°C, a combustion reference state of 25°C, and 101.325 kPa), a specific gravity d, a carbon dioxide concentration $x_{CO2}$, and a hydrogen concentration $x_{H2}$ as input parameters to calculate a large number of items as intermediate data, the items including a molar fraction $x_{CH}$ of a hydrocarbon gas, a molar fraction $x_{N2}$ of nitrogen, a molar fraction $x_{CO}$ of carbon monoxide, a molar heating value $H_{CH}$ of an equivalent hydrocarbon gas, a molar mass $M_{CH}$ of an equivalent hydrocarbon gas, a second virtual conversion coefficient (Tn = 273.15 K) Bn, a molar density $\rho m, n$ under standard conditions, a mass density $\rho n$ under standard conditions, and the gross heating value Hs. After the calculation, the compression factor Z is calculated using these intermediate data.

In general, a combustion speed MCP of city gas is typically calculated from the composition analysis values of gas chromatography using a (Pa) formula shown below.

$$\mathtt{MCP = (1 - K) \times \{\Sigma(Si \cdot fi \cdot Ai)\}/\{\Sigma(fi \cdot Ai)\} \quad (Pa)}$$

Si: combustion speed of flammable gas component i in the gas
fi: factor relating to each flammable gas component i in the gas
Ai: volume percentage of each flammable gas component i in the gas
K: attenuation factor with a value calculated by the following equation:

$$\mathtt{K = \{\Sigma Ai/(\Sigma \alpha i \cdot Ai)\} \times \{(2.5CO_2 + N_2 - 3.77O_2)/}$$

$$\mathtt{(100 - 4.77O_2) + [(N_2 - 3.77O_2)/(100 - 4.77O_2)]^2\}}$$

αi: correction coefficient for flammable gas component i in the gas
$CO_2$, $N_2$, $O_2$: volume percentage of each component in the gas

Citation List

Patent Literature

PTL 1: Japanese Patent No. 6402387 Further prior art documents are WO 2020/148927 A1 showing a composition analysis device and composition analysis method, JP 2021 004891 A showing a methane concentration calculation method and methane concentration measurement device and WO 2016/104270 A1 showing a method for calculating methane number and device for determining methane number.

Summary of Invention

Technical Problem

However, for different physical property values (for example, a compression factor, and a combustion speed), parameters used for their calculations are often different from one another, necessitating the use of methods for acquiring the respective parameters. Naturally, when a large number of parameters are used as in the case of calculating the compression factor, the procedures and devices involved in the calculation become complicated.

In the case of the compression factor in particular, the pressure P and the temperature T are used as initial input data in the calculation methods according to ISO12213-2 and ISO12213-3. However, under conditions of different temperatures and pressures, it is necessary to re-calculate starting from all of the input data required for the respective calculation methods in order to calculate the compression factor. This necessity causes a problem of inconvenience. Also there is a problem in that when the temperature or pressure is not measurable, numerical values relating to the compression factor cannot be acquired.

These problems also exist in the calculation of other physical property values (for example, the combustion speed). The combustion speed in particular is calculated using the analytical values of a gas chromatography device, which is a large and expensive device. As a result, the calculation is costly and is not easily performed.

Thus, pertaining to the calculation of a plurality of types of physical property values in particular, studies on simple and relatively low-cost means and techniques have not made significant progress so far.

The present invention has been made in view of the above-described problems, and it is an object of the present invention to provide a calculation device, a calculation method, and a program that can perform easy and relatively low-cost calculation of a plurality of types of physical property values relating to combustion gases.

Solution To Problem

The present invention relates to a calculation device according to claim 1.

The present invention also relates to a computer-implemented calculation method according to claim 9.

The present invention also relates to a program for causing a computer to execute the calculation method described above. Advantageous Effects of Invention

The present invention can provide a calculation device, a calculation method, and a program that can perform easy and relatively low-cost calculation of a plurality of types of physical property values relating to combustion gases.

Brief Description of Drawings

[Fig. 1] Fig. 1 is a block diagram showing an example of a calculation device according to a present embodiment.
[Fig. 2] Fig. 2 is a block diagram showing an example of a calculation device according to the present embodiment.
[Fig. 3] Fig. 3 includes flowcharts showing an example of a calculation method according to the present embodiment.
[Fig. 4] Fig. 4 includes flowcharts describing a calculation method of a compression factor in the present embodiment.
[Fig. 5] Fig. 5 includes flowcharts describing a calculation method of a combustion speed in the present embodiment.
[Fig. 6] Fig. 6 is a graph showing an example of the relation between the heating value and the compression factor of gases.
[Fig. 7] Fig. 7 shows the results of verification of normalized compression factors calculated by the present embodiment.
[Fig. 8] Fig. 8 is a graph comparing correction coefficients that correct the influence of miscellaneous gases.
[Fig. 9] Fig. 9 includes graphs comparing the compression factors calculated by a conventional method and compression factors calculated by the method of the present embodiment.
[Fig. 10] Fig. 10 includes graphs comparing the compression factors calculated by a conventional method and compression factors calculated by the method of the present embodiment.
[Fig. 11] Fig. 11 includes graphs comparing the compression factors calculated by a conventional method and compression factors calculated by the method of the present embodiment.
[Fig. 12] Fig. 12 includes graphs comparing the compression factors calculated by a conventional method and compression factors calculated by the method of the present embodiment.
[Fig. 13] Fig. 13 includes graphs comparing the compression factors calculated by a conventional method and compression factors calculated by the method of the present embodiment.

[Fig. 14] Fig. 14 includes graphs comparing the compression factors calculated by a conventional method and compression factors calculated by the method of the present embodiment.

[Fig. 15] Fig. 15(A) is a graph showing the relation between a basic heating value (calculated value) and a combustion speed of a plurality of natural gases by an MCP calculation formula according to the conventional method, and Fig. 15(B) is a graph indicating the relation between a change amount in combustion speed and the basic heating value of natural gases containing the miscellaneous gas components.

[Fig. 16] Fig. 16 includes graphs comparing the combustion speed calculated by the conventional method and the combustion speed calculated by the method of the present embodiment.

Description Of Embodiments

Embodiments of the present invention will be described below with reference to the accompanying drawings. First, a calculation device 1 according to an embodiment of the present invention is a device configured to calculate a value relating to a combustible target gas containing the miscellaneous gas components. Here, examples of the "value relating to the combustible target gas" in the present embodiment include values (physical property values, and factors) relating to the combustion of the target gas (combustible gas), and values (physical property values, and factors) relating to the states of the combustible gas. Here, a description will be given of the case where, for example, the value (physical property values, and factors) relating to the combustion of combustible gas is a basic heating value or a value relating to the basic heating value or a combustion speed, and the value (physical property values, and factors) relating to the states of the combustible gas is a value relating to the compression factor.

The target gas of the present embodiment is a natural gas that is just produced from gas fields or a biogas. Examples of the target gas include a natural gas containing miscellaneous gas (noncombustible gas) components. Specifically, the target gas is a gas containing a first gas (a combustible gas) as a main component and a second gas (a miscellaneous gas, a noncombustible gas) that is a measurement error component. More specifically, examples of the first gas include paraffin-based hydrocarbon gases (for example, a methane gas ($CH_4$), an ethane gas ($C_2H_6$), a propane gas ($C_3H_8$), and a butane gas (n-$C_4H_{10}$), which have a specific correspondence relation between their heating values and refractive indexes and between their heating values and densities (specifically, the densities are inversely proportional to the heating values, and the refractive indexes are proportional to the heating values).

The second gas is a gas as the miscellaneous gas components, which has no specific correspondence relation between its heating value and its refractive index and between its heating value and its density. For example, the miscellaneous gas components are a gas mainly containing (a mixture of) nitrogen ($N_2$) and carbon dioxide ($CO_2$). The "gas mainly containing nitrogen (N2) and carbon dioxide (CO2)" is a gas that does not contain noncombustible gas components other than N2 and CO2 or a hydrogen gas and a carbon monoxide gas components or that contains a very small amount of above gas components that are small enough to be negligible in terms of the calculation error.

The heating value Hs of a natural gas in the following description is standardized as a gross heating value MJ/m$^3$ (measurement reference state temperature of 0°C, combustion reference state of 0°C, 101.325 kPa). The units of concentration of various gases are standardized as molar concentration [mol%].

<Calculation device>

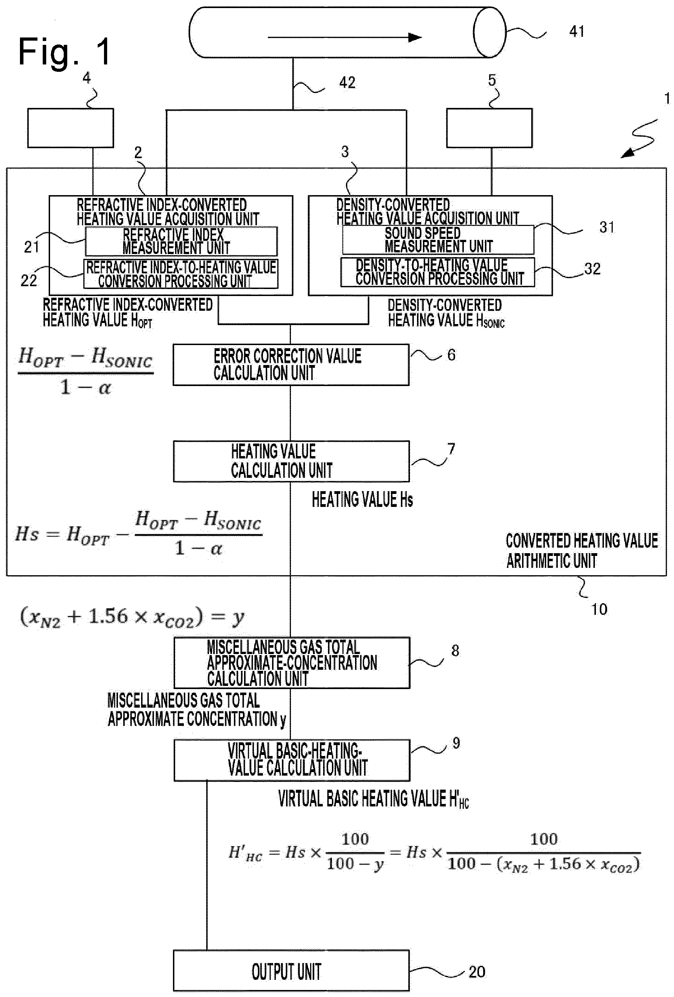

Fig. 1 is a block diagram schematically showing an example of the configuration of the calculation device 1 according to a first embodiment. For example, the calculation device 1 of the present embodiment is a device configured to calculate values relating to the target gas that circulates through a gas pipeline 41 in the direction of an arrow in Fig. 1. The calculation device 1 includes a converted heating value arithmetic unit 10, a miscellaneous gas total approximate-concentration calculation unit 8, a virtual basic-heating-value calculation unit 9, and an output unit 20.

The converted heating value arithmetic unit 10 includes a refractive index-converted heating value acquisition unit 2, a density-converted heating value acquisition unit 3, an error correction value calculation unit 6, and a heating value calculation unit 7. The converted heating value arithmetic unit 10 is a unit configured to arithmetically operate and output a heating value (converted heating value) of the target gas while taking into account the error caused by miscellaneous gas components. This arithmetic occurs on the basis of a converted heating value that is converted from a refractive index of the target gas (including miscellaneous gas components) and a converted heating value that is converted from density.

The gas pipeline 41 is connected to the refractive index-converted heating value acquisition unit 2 and the density-converted heating value acquisition unit 3 through a gas flow channel 42, and the target gas in the gas pipeline 41 is thus supplied to the refractive index-converted heating value acquisition unit 2 and the density-converted heating value acquisition unit 3. For example, the calculation device 1 is disposed inside an explosion-proof container (not shown).

The refractive index-converted heating value acquisition unit 2 is a device (for example, an interferometer, a refractive index calorimeter, etc.) configured to detect, for example, a difference in the refractive index of light between the target gas and a standard gas such as air. This difference is detected as a displacement of interference fringes, and the detection occurs on the basis of an output signal (a voltage applied to the refractive index-converted heating value acquisition unit 2) from an external device (such as a power source device) 4. Based on the displacement amount of the interference fringes, a refractive index-converted heating value $H_{OPT}$ of the target gas is acquired.

For example, the refractive index-converted heating value acquisition unit 2 includes a refractive index measurement unit 21 (for example, a light sensor) configured to measure the refractive index of the target gas, and a refractive index-to-heating value conversion processing unit 22 having a function of obtaining the refractive index-converted heating value $H_{OPT}$ on the basis of the value of the refractive index measured by the refractive index measurement unit 21.

The refractive index-to-heating value conversion processing unit 22 has a prescribed correlation expression based on the refractive index and the heating value acquired in advance for gases (gases not containing miscellaneous gas (noncombustible gas) components) composed only of the combustible gas components that serve as standards (paraffin-based hydrocarbon gases). The refractive index-to-heating value conversion processing unit 22 then calculates the refractive index-converted heating value $H_{OPT}$ of the target gas by applying the value of the actually measured refractive index of the target gas to the correlation expression. This calculation is made with the assumption that the value of the actually measured refractive index of the target gas is the refractive index of the combustible gas.

For example, the density-converted heating value acquisition unit 3 is a device configured to measure the sound speed of the target gas on the basis of an output signal (a voltage applied to the density-converted heating value acquisition unit 3) from an external device (such as a power supply unit) 5 to acquire a density-converted heating value $H_{SONIC}$ of the target gas.

The density-converted heating value acquisition unit 3 includes a sound speed measurement unit (for example, a sound speed sensor) 31 configured to measure propagation speed of sound waves in the target gas (the sound speed of the target gas), and a density-to-heating value conversion processing unit configured to calculate the density of the target gas (= elastic modulus/ sound speed$^2$) on the basis of the sound speed measured by the sound speed measurement unit 31 to obtain the density-converted heating value $H_{SONIC}$.

The density-to-heating value conversion processing unit has a prescribed correlation expression based on the density (sound speed) and the heating value acquired in advance for gases (gases not containing miscellaneous gas (noncombustible gas) components) composed only of the combustible gas components that serve as standards (paraffin-based hydrocarbon gases). The density-to-heating value conversion processing unit calculates the density-converted heating value $H_{SONIC}$ of the target gas by applying the value of the actually measured density of the target gas to the correlation expression on the assumption that the actually measured value of the density is the density of the combustible gas.

The error correction value calculation unit 6 calculates a value (an error correction value: a second term of the right side of the formula (7) described later) that corrects an error (an error caused by miscellaneous gases) in the calculation of the heating value, the error being caused by the miscellaneous gases (a nitrogen gas and a carbon dioxide gas) contained in the target gas.

The heating value calculation unit 7 is a unit that has a converted heating value arithmetic formula (the formula (7) described later) and is configured to calculate a heating value Hs of the target gas. This calculation is performed by a prescribed arithmetic operation on the basis of converted heating values obtained from the refractive index and the density of the target gas (the refractive index-converted heating value $H_{OPT}$ and the density-converted heating value $H_{SONIC}$), respectively, and on the basis of the error correction value. The prescribed arithmetic operation is an arithmetic operation (arithmetic operation of a converted heating value) uniquely developed by the applicant of the present invention. The arithmetic operation is hereinafter referred to as Opt-Sonic arithmetic operation.

The miscellaneous gas total approximate-concentration calculation unit 8 is a unit, having a miscellaneous gas total approximate-concentration calculation formula (the formula (8) described later), configured to calculate a total approximate concentration of the miscellaneous gas components in the target gas (a miscellaneous gas total approximate concentration y) on the basis of the refractive index-converted heating value $H_{OPT}$ and the density-converted heating value $H_{SONIC}$. The target gas handled in the present embodiment mainly contains a nitrogen gas and a carbon dioxide gas as miscellaneous gas components. Even when the individual concentrations of these gases are unknown, the concentrations are replaced with a total concentration that allows an approximation error, and various arithmetic operations are performed with the total concentration. The total concentration of the miscellaneous gases that allows an approximation error is the miscellaneous gas total approximate concentration y. The miscellaneous gas total approximate-concentration calculation unit 8 can calculate the total approximate concentration of the miscellaneous gas components (a nitrogen gas and a carbon dioxide gas) (the miscellaneous gas total approximate concentration y) on the basis of the refractive index-converted heating value $H_{OPT}$ and the density-converted heating value $H_{SONIC}$.

The virtual basic-heating-value calculation unit 9 has a virtual basic-heating-value calculation formula (the formula (9) described later). When the concentration of miscellaneous gases (a nitrogen gas and a carbon dioxide gas) is unknown, the virtual basic-heating-value calculation unit 9 assumes that the total concentration of the miscellaneous gases is the miscellaneous gas total approximate concentration y. Then, the virtual basic-heating-value calculation unit 9 obtains by arithmetic operation a basic heating value H'$_{HC}$ (i.e., a heating value of only the combustible gas component in the target gas) of the target gas when the miscellaneous gas components based on the assumption are removed.

Here, the "basic heating value" of the target gas refers to the heating value when the target gas is composed only of combustible gases (paraffin-based hydrocarbon gases). When the target gas contains miscellaneous gas components, they are removed and then the heating value is measured (calculated). Therefore (as will be described later), it is originally necessary to measure the concentration of the miscellaneous gas components. With this regard, the virtual basic-heating-value calculation unit 9 in the present invention calculates a value equivalent to the basic heating value by arithmetic operation, even when the concentration of the miscellaneous gas components is unknown. Specifically, on the basis of the heating value Hs of the target gas calculated by the heating value calculation unit 7 and the miscellaneous gas total approximate concentration y calculated by the miscellaneous gas total approximate-concentration calculation unit 8, the value corresponding to the basic heating value of the target gas when the miscellaneous gas components are assumed to be removed (i.e., the heating value of only the combustible gas component in the target gas) is obtained by arithmetic operation. In the present embodiment, the value equivalent to the basic heating value calculated by the virtual basic-heating-value calculation unit 9 is called the virtual basic heating value H'$_{HC}$. The output unit 20 outputs the virtual basic heating value H'$_{HC}$.

In general, measuring the concentration of the miscellaneous gas components is a large-scale and complicated task involving, for example, use of gas chromatography. In contrast, according to the calculation device 1 of the present embodiment, when the refractive index and the sound speed of the target gas (containing miscellaneous gas components) can be measured as inputs, the heating value (virtual basic heating value H'$_{HC}$) of only the combustible gas in the target gas can be calculated even in the case where the concentration of the miscellaneous gas components is unknown.

The virtual basic heating value H'$_{HC}$ output as a result of calculation by the calculation device 1 is used as a substitute of the basic heating value of the target gas. In other words, the virtual basic heating value H'$_{HC}$ can be used to calculate various physical property values (for example, a compression factor, a combustion speed, etc.) that can be calculated using the basic heating value. While the reason why such substitution is possible will be described in detail later, the refractive index measurement unit (refractive index calorimeter) and the sound speed measurement unit (sound speed sensor) are generally small and relatively inexpensive devices. Therefore, as compared to the conventional methods (calculation of the compression factor according to ISO12213-3 and calculation of the combustion speed by the (Pa) formula), the physical property values relating to the natural gas can be calculated simply and easily.

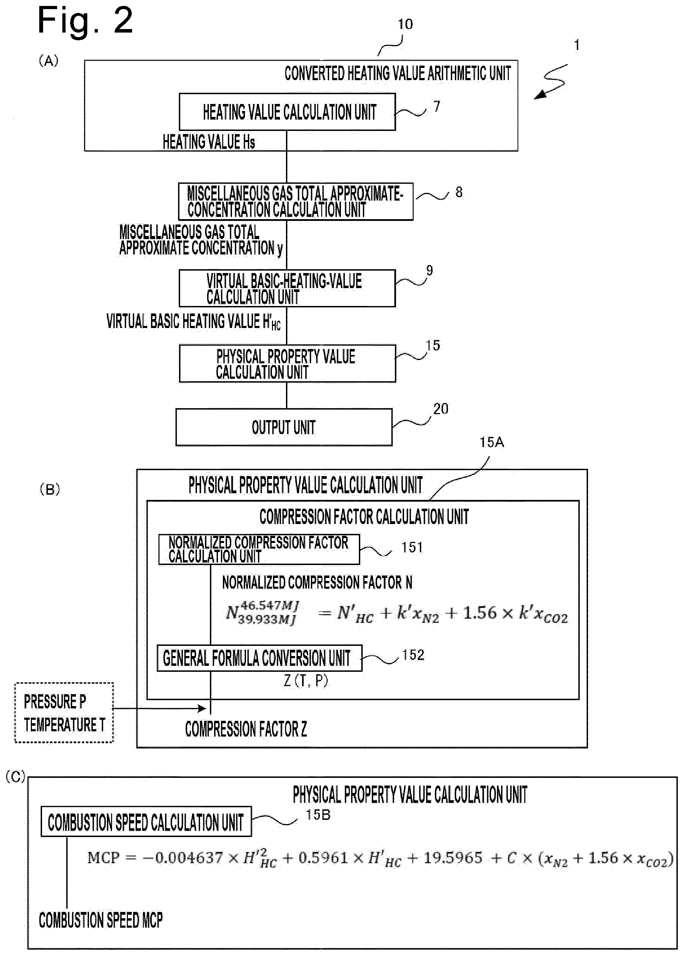

Fig. 2 includes schematic views showing an example of the calculation device 1 including a physical property value calculation unit 15 configured to calculate various physical property values relating to the target gas on the basis of the virtual basic heating value H'$_{HC}$. Component members identical to those shown in Fig. 1 are designated by identical reference signs to omit the description thereof. The component members other than the physical property value calculation unit 15 are similar to the component members in Fig. 1. Fig. 2(A) is a block diagram showing the overall configuration of the calculation device 1, and Figs. 2(B) and 2(C) are block diagrams showing an example of the physical property value calculation unit 15.

Fig. 2(B) is an example of the case where the physical property value calculation unit 15 is a compression factor calculation unit 15A configured to calculate a compression factor. The compression factor calculation unit 15A includes a unit (normalized compression factor calculation unit) 151 configured to calculate a compression factor N that is normalized (hereinafter referred to as a "normalized compression factor"), and a general formula conversion unit 152 configured to convert the normalized compression factor N into a general formula.

Although described later in detail, the normalized compression factor N is a normalized expression of a gas compression factor, which is originally expressed as a function of the temperature T and the pressure P and is normalized so as to eliminate the influence of these parameters. The normalized compression factor calculation unit 151 has a normalized compression factor calculation formula (the formula (10) described later) and calculates the normalized compression factor N on the basis of the virtual basic heating value H'$_{HC}$ and the miscellaneous gas total approximate concentration y. The normalized compression factor calculation unit 151 corrects an error caused by using the miscellaneous gas total approximate concentration y and calculates the normalized compression factor N (this correction will be described later). The compression factor calculation unit 15A receives the input of the pressure P and the temperature T, generalizes the normalized compression factor N by the general formula conversion unit 152, and calculates a compression factor Z under the conditions of the pressure P and the temperature T to be required.

Fig. 2(C) is an example of the case where the physical property value calculation unit 15 is a combustion speed calculation unit 15B configured to calculate a combustion speed MCP. The combustion speed calculation unit 15B has a combustion speed calculation formula (the formula (15) described later) and calculates the combustion speed MCP on the basis of the virtual basic heating value H'$_{HC}$ and the miscellaneous gas total approximate concentration y. The combustion speed calculation unit 15B corrects an error caused by using the miscellaneous gas total approximate concentration y and calculates the combustion speed MCP (this correction will be described later).

Thus, the calculation device 1 of the present embodiment acquires two variables of the refractive index-converted heating value H$_{OPT}$ and the density-converted heating value H$_{SONIC}$, and uses the Opt-Sonic arithmetic operation to calculate the virtual basic heating value $H'_{HC}$. The calculation device 1 also approximately calculates a plurality of physical property values relating to a natural gas by using the virtual basic heating value $H'_{HC}$.

In the case where a physical property value is a value that can originally be calculated using the basic heating value of a natural gas in particular, the virtual basic heating value $H'_{HC}$ can be used as it is as a substitute of the basic heating value. For example, in a first method according to the method originally conceived by the applicant of the present invention as will be described later, the physical property values, such as the compression factor and the combustion speed, can be calculated by a polynomial formula (three input values (input parameters)) of three variables, i.e., the basic heating value of a natural gas, and the concentrations of a carbon dioxide gas and a nitrogen gas that are miscellaneous gas components. Therefore, the physical property values can be calculated more easily and with a configuration simpler compared with using the conventional calculation method according to ISO12213-3 or the (Pa) formula. In a second method, according to the present embodiment described above, the physical property values, such as the compression factor and the combustion speed, can be calculated with two input values, such as the refractive index and the density of the target gas, by using the virtual basic heating value as a substitute of the basic heating value. In other words, in calculation of the same physical property value, the second method can further reduce the number of input parameters as compared to the first method. In addition, the refractive index and the density of the target gas can be measured using small and relatively inexpensive devices, and therefore the plurality of physical property values relating to the natural gas, which can be calculated using the basic heating value $H_{HC}$, can be calculated simply and easily.

<Calculation method and program>

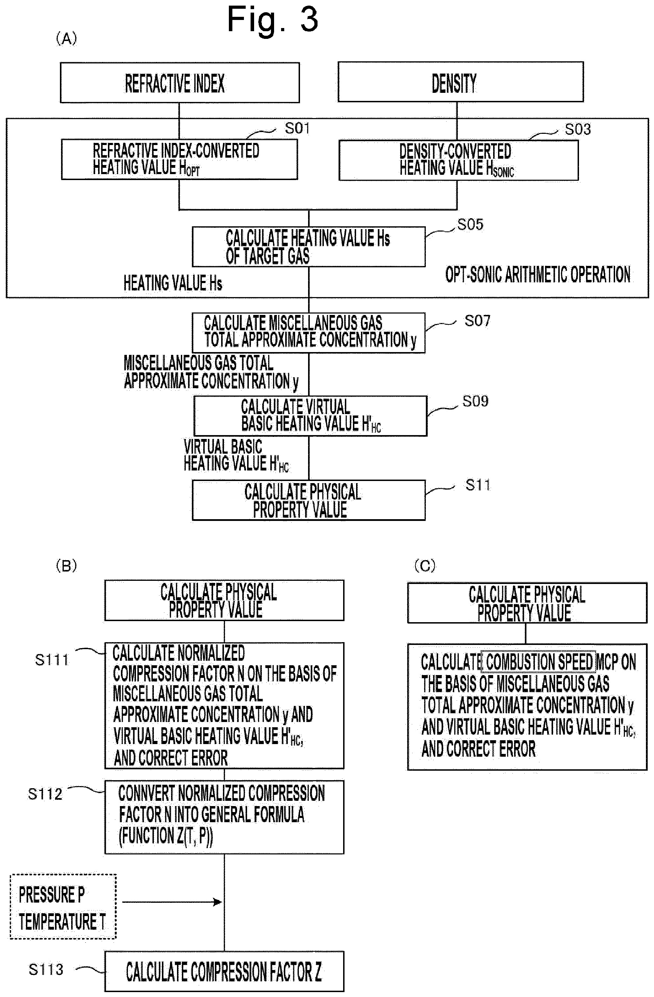

With reference to Fig. 3, the embodiment of the present invention provides a method of calculating a physical property value relating to a combustible target gas containing miscellaneous gas components. Specifically, as shown in Fig. 3(A), the method relates to a calculation method, including: a step of measuring the refractive index of the target gas and acquiring the refractive index-converted heating value $H_{OPT}$ (step S01); a step of measuring the density of the target gas and acquiring the density-converted heating value $H_{SONIC}$ (step S03); a step of calculating the heating value Hs of the target gas by the Opt-Sonic arithmetic operation on the basis of the refractive index-converted heating value $H_{OPT}$ and the density-converted heating value $H_{SONIC}$ (step S05); a step of calculating the approximate concentration of the miscellaneous gas components (a miscellaneous gas total approximate concentration y) by a miscellaneous gas total approximate concentration calculation formula on the basis of the refractive index-converted heating value $H_{OPT}$ and the density-converted heating value $H_{SONIC}$ (step S07); and a step of calculating the virtual basic heating value $H'_{HC}$ of the target gas by a virtual basic heating value calculation formula on the basis of the heating value Hs of the target gas and the miscellaneous gas total approximate concentration y (step S09). The calculated virtual basic heating value $H'_{HC}$ can be used, for example, to calculate various physical property values that can be acquired on the basis of the basic heating value $H_{HC}$ of the target gas (step S11).

As shown in Fig. 3(B), when the compression factor Z is calculated as an example of the physical property value, the normalized compression factor N is calculated on the basis of the miscellaneous gas total approximate concentration y calculated in step S07 in Fig. 3(A) and the virtual basic heating value $H'_{HC}$ calculated in step S09 in Fig. 3(A). In the calculation of the normalized compression factor N, an error caused by using the miscellaneous gas total approximate concentration y is corrected (error correction is performed) in accordance with miscellaneous gas components (a carbon dioxide gas concentration $x_{CO2}$) (step S111).

The normalized compression factor N is then converted into a general formula for the compression factor (function Z(T, P)) with the temperature T and the pressure P as parameters (step S112).

Then, given values to be input as the temperature T and the pressure P are input into the general formula (function Z(T, P)) to calculate the compression factor Z (step S113).

As shown in Fig. 3(C), when the combustion speed is calculated as an example of the physical property value, the combustion speed is calculated by a combustion speed calculation formula on the basis of the miscellaneous gas total approximate concentration y and the virtual basic heating value $H'_{HC}$. In the calculation of the combustion speed, an error caused by using the miscellaneous gas total approximate concentration y is corrected (error correction is performed).

The present embodiment of the present invention also provides a program for causing a computer to execute the above-described calculation method.

<Calculation method of physical property value that can reduce number of input parameters>

Next, as another embodiment of the present invention, a technique will be described that can reduce the number of input parameters in the calculation of the compression factor of a target gas and in the calculation method of the compression factor, as compared to the conventional methods (for example, the calculation method according to ISO12213-3 and the method according to the (Pa) formula). For example, when the target gas is a natural gas (paraffin-based hydrocarbon gas) that contains miscellaneous gas components mainly composed of a nitrogen gas and a carbon dioxide gas (that does not contain, or contains a negligible amount of, hydrogen and carbon monoxide), the compression factor and the combustion speed can be calculated by following methods.

<Calculation method of compression factor>

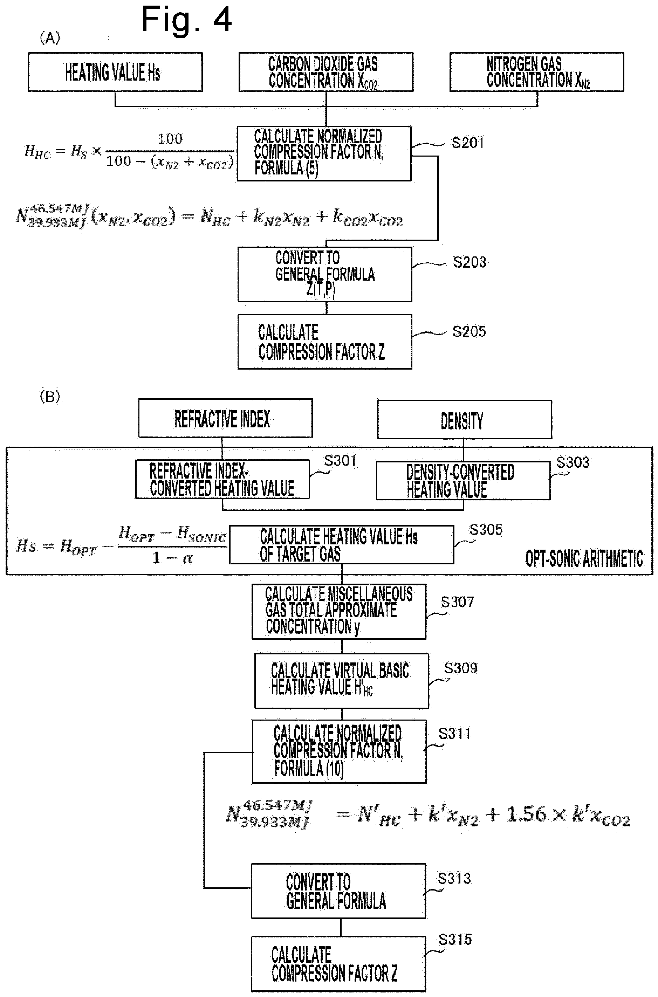

Fig. 4(A) is a flowchart showing a first method of calculating the compression factor Z. Fig. 4(B) is a flowchart showing a second method of calculating the compression factor Z.

(Compression factor calculation / First method)

The first method is a method of calculating the compression factor Z using three input parameters, i.e., the heating value Hs of a natural gas, a carbon dioxide gas concentration $x_{CO2}$, and a nitrogen gas concentration $x_{N2}$. The method includes: a step of calculating the normalized compression factor N by inputting into a normalized compression factor calculation formula (the formula (5) described later) the heating value Hs of a natural gas, the carbon dioxide gas concentration $x_{CO2}$, and the nitrogen gas concentration $x_{N2}$ (step S201); a step of converting the normalized compression factor N into a general formula for the compression factor (function Z(T, P)) with the pressure P and the temperature T as parameters (step S203); and a step of calculating the compression factor Z under the conditions of the pressure P and the temperature T to be required (step S205).

The compression factor Z calculated using the calculation method according to ISO12213-3 is a value expressed as a function of a temperature T, a pressure P, a heating value Hs (a gross heating value converted to a measurement reference state temperature of 0°C, a combustion reference state of 25°C, and 101.325 kPa), a specific gravity d, and a carbon dioxide concentration $x_{CO2}$ (function Z(T, P, Hs, d, $x_{CO2}$)). The applicant of the present invention tried to normalize compression factors $Z_A$(T, P, Hs, d, $x_{CO2}$) and $Z_B$(T, P, Hs, d, $x_{CO2}$) of two different types of gases A and B so that their solutions are equal to 0 and -1, respectively. As a result, the applicant found that the compression factor Z can be expressed approximately by a polynomial formula of the basic heating value $H_{HC}$ based on the heating value Hs of a natural gas, the carbon dioxide gas concentration $x_{CO2}$, and the nitrogen gas concentration $x_{N2}$ (the formula (5) described later). The value obtained by the polynomial formula is the normalized compression factor N.

In the conventional technique (the calculation method according to ISO12213-3), the pressure P and the temperature T are used as initial input data. When the compression factor Z is calculated under the conditions of different temperatures and pressures, re-calculation must be performed starting from all the input data for the respective calculation methods, causing inconvenience.

According to the first method of compression factor calculation, three input parameters, the heating value Hs of a natural gas, and the carbon dioxide gas concentration $x_{CO2}$ and the nitrogen gas concentration $x_{N2}$ in the natural gas, are used to calculate the normalized compression factor N independent of the temperature T and the pressure P. In this case, the formulas (5) and (6) described later are used. The normalized compression factor N is further converted into a general formula (function Z(T, P)) with the temperature T and the pressure P as parameters. In other words, when the compression factor Z is calculated under the conditions of different temperatures and pressures, various compression factors Z can be calculated by changing only the input parameters, the temperature T and the pressure P in the general formula (function Z(T, P)).

Therefore, the first method of the compression factor calculation includes: calculating the basic heating value Hs on the basis of the heating value Hs of a natural gas, and the carbon dioxide gas concentration $x_{CO2}$ and the nitrogen gas concentration $x_{N2}$ in the natural gas; calculating the normalized compression factor N on the basis of the basic heating value Hs, and the carbon dioxide gas concentration $x_{CO2}$ and the nitrogen gas concentration $x_{N2}$ in the natural gas; and calculating the compression factor Z on the basis of the normalized compression factor N.

(Compression factor calculation/ second method)

With reference to Fig. 4(B), a second method of calculating the compression factor Z will be described. The second method is a method to further reduce the input parameters and calculate the compression factor Z on the basis of two input parameters, the refractive index and the density of a natural gas. Specifically, the second method includes: a step of calculating the refractive index-converted heating value $H_{OPT}$ on the basis of the refractive index (step S301); a step of calculating the density-converted heating value $H_{SONIC}$ on the basis of the density (step S303); a step of calculating the heating value Hs of a natural gas by the Opt-Sonic arithmetic formula on the basis of the refractive index-converted heating value $H_{OPT}$ and the density-converted heating value $H_{SONIC}$ (step S305); a step of calculating the miscellaneous gas total approximate concentration y (step S307); a step of calculating the virtual basic heating value $H'_{HC}$ (step S309); a step of calculating the normalized compression factor N (step S311); a step of converting the normalized compression factor N into a general formula for the compression factor (function Z(T, P)) with the temperature T and the pressure P as parameters (step S313); and a step of calculating the compression factor Z by inputting the pressure P and the temperature T required for the general formula (function Z(T, P)) (step S315).

The second method of the compression factor calculation corresponds to the calculation method for obtaining the compression factor Z as a physical property value described in Figs. 3(A) and 3(B) above. According to the second method, the normalized compression factor N independent of the temperature T and the pressure P can be calculated with two input parameters, the refractive index and the density of a natural gas. In this case, the formula (10) described later is used. Then, the normalized compression factor N is converted into a general formula (function Z(T, P)) with the temperature T and the pressure P serving as parameters. In other words, when the compression factor Z is calculated under the conditions of different temperatures and pressures, various compression factors Z can be calculated by changing only the input parameters, the temperature T and the pressure P in the function Z(T, P)).

It is also possible to configure compression factor calculation devices capable of executing the above-described methods. Although illustration is omitted, a compression factor calculation device capable of executing the first method of compression factor calculation includes: an acquisition unit capable of acquiring each of the heating value Hs of a natural gas, the carbon dioxide gas concentration $x_{CO2}$, and the nitrogen gas concentration $x_{N2}$; a unit configured to calculate the basic heating value Hs on the basis of the heating value Hs, the carbon dioxide gas concentration $x_{CO2}$, and the nitrogen gas concentration $x_{N2}$; a normalized compression factor calculation unit that has a normalized compression factor calculation formula; a conversion unit for conversion to a general formula for the compression function (function Z(T, P)); and an output unit configured to output the calculated compression factor Z. It is also possible to configure a program for causing a computer to execute the above-described method.

A compression factor calculation device capable of executing the second method of compression factor calculation and a program are the same as the calculation device 1 described in Figs. 2(A) and 2(B) described above, and so the description thereof is omitted.

<Calculation method of combustion speed>

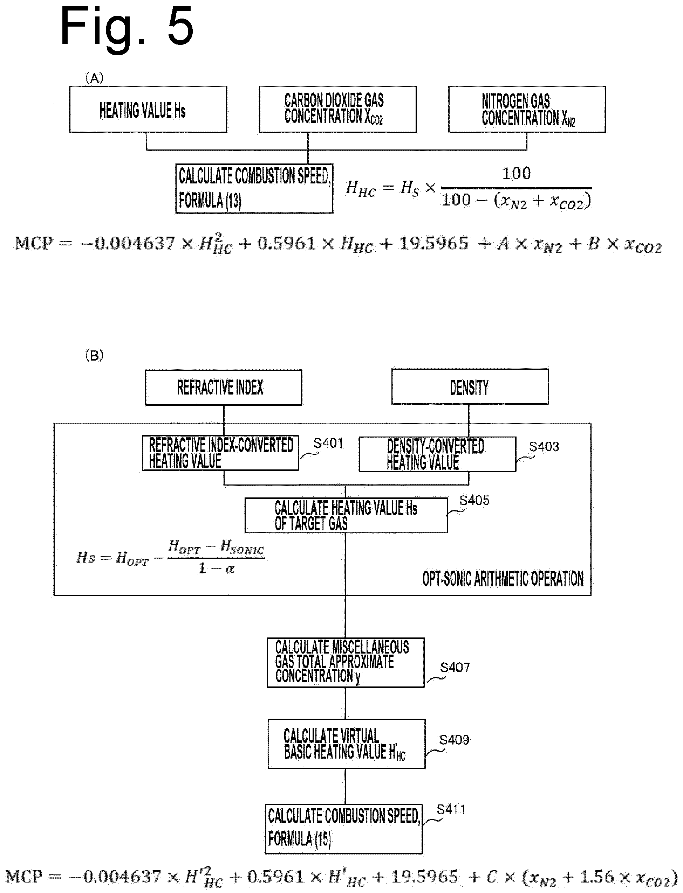

Next, with reference to Figs. 5, a calculation method of the combustion speed will be described. Fig. 5(A) is a flowchart showing a first method of calculating the combustion speed. Fig. 5(B) is a flowchart showing a second method of calculating the combustion speed.

(Combustion speed calculation / First method)

The first method of calculating the combustion speed is a method of calculating the combustion speed MCP using three input parameters, i.e., the heating value Hs of a natural gas, the carbon dioxide gas concentration $x_{CO2}$, and the nitrogen gas concentration $x_{N2}$ by using a combustion speed calculation formula shown in the formula (13) described later (and the formula (14)).

Specifically, the first method of calculating the combustion speed includes: calculating the basic heating value Hs on the basis of the heating value Hs of a natural gas, and the carbon dioxide gas concentration $x_{CO2}$ and the nitrogen gas concentration $x_{N2}$ in the natural gas; and calculating the combustion speed MCP on the basis of the basic heating value Hs, and the carbon dioxide gas concentration $x_{CO2}$ and the nitrogen gas concentration $x_{N2}$ in the natural gas.

<Combustion speed calculation / Second method>

With reference to Fig. 5(B), the second method of calculating the combustion speed MCP will be described. The second method is a method to further reduce the input parameters and calculate the combustion speed MCP on the basis of two input parameters, the refractive index and the density of a natural gas. Specifically, the second method includes: a step of calculating the refractive index-converted heating value $H_{OPT}$ on the basis of the refractive index (step S401); a step of calculating the density-converted heating value $H_{SONIC}$ on the basis of the density (step S403); a step of calculating the heating value Hs of a natural gas by the Opt-Sonic arithmetic formula on the basis of the refractive index-converted heating value $H_{OPT}$ and the density-converted heating value $H_{SONIC}$ (step S405); a step of calculating the miscellaneous gas total approximate concentration y (step S407); a step of calculating the virtual basic heating value $H'_{HC}$ (step S409); and a step of calculating the combustion speed MCP by a combustion speed calculation formula shown in the formula (15) described later (step S411).

The second method corresponds to the calculation method of obtaining the combustion speed as a physical property value described in Figs. 3(A) and 3(C) above. According to the second method, the combustion speed MCP can be calculated using two input parameters, the refractive index and the density of a natural gas.

It is also possible to configure combustion speed calculation devices capable of executing the above-described methods. Although illustration is omitted, a combustion speed calculation device capable of executing the first method of calculating the combustion speed includes: an acquisition unit capable of acquiring each of the heating value Hs of a natural gas, the carbon dioxide gas concentration $x_{CO2}$, and the nitrogen gas concentration $x_{N2}$; a unit configured to calculate the basic heating value Hs on the basis of the heating value Hs, the carbon dioxide gas concentration $x_{CO2}$, and the nitrogen gas concentration $x_{N2}$; a combustion speed calculation unit having a combustion speed calculation formula; and an output unit configured to output the calculated combustion speed MCP. It is also possible to configure a program for causing a computer to execute the above-described method.

A combustion speed calculation device capable of executing the second method of calculating the combustion speed and a program are the same as the calculation device 1 described in Figs. 2(A) and 2(C) above, and so the description thereof is omitted.

A detailed description will now be given of simulations and consideration on the basis of which the applicant of the present invention could conceive the configuration of the respective embodiments, and the calculation formulas (functions) derived on the basis of these simulations and consideration.

<Normalized compression factor N>

A description will be given of the concept of the normalized compression factor N and the calculation method thereof as conceived by the applicant of the present invention. A natural gas in the following description is a gas having a paraffin-based hydrocarbon gas as a main component.

First, the calculation method of the compression factor Z according to ISO12213-3 was used to examine the characteristics of a compression factor $Z_{HC}$ of a natural gas composed only of paraffin-based hydrocarbon gases having $CH_4$ as a main component. In this case, the compression factor $Z_{HC}(P,T)$ under the conditions of the pressure P and the temperature T changes depending on a heating value $H_{HC}$ regardless of its composition. In other words, as shown in the formula (1) shown below, the compression factor $Z_{HC}(P,T)$ can be expressed as a polynomial formula (a function determined by the pressure P and the temperature T) of the heating value $H_{HC}$. [Mathematical formula 1]

$$Z_{HC}(P,T) = \sum_{h=0}^{3} a_h(P,T) H_{HC}^h \qquad (1)$$

Here, $a_h$ (a3, a2, a1, a0) in the formula (1) represent coefficients of respective polynomial terms.

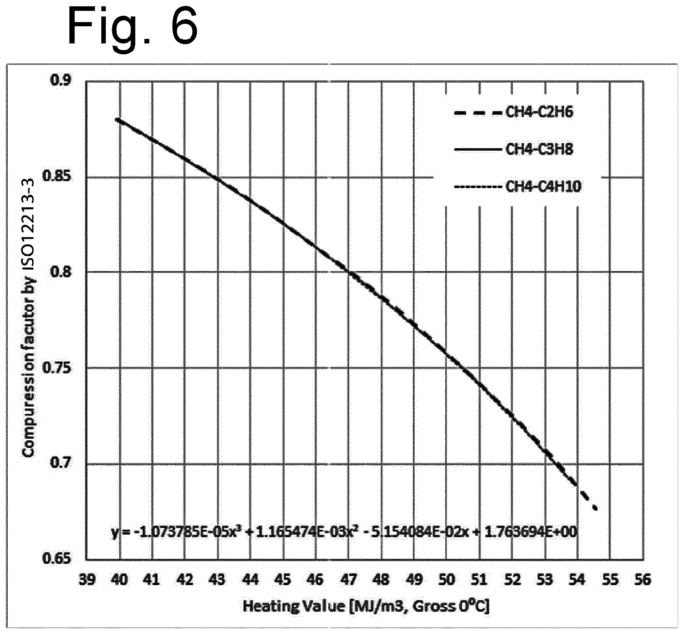

Fig. 6 is a graph showing an example of the relation between the heating value $H_{HC}$ and the compression factor $Z_{HC}$ of gases expressed by the formula (1). The horizontal axis represents the heating value $H_{HC}$ of the gases composed only of paraffin-based hydrocarbon gases, and the vertical axis represents the compression factor $Z_{HC}$ under the conditions of a pressure of 7 MPa and a temperature of 20°C.

Here, as the paraffin-based hydrocarbon gases, three types of mixed gases, containing methane ($CH_4$), ethane ($C_2H_6$), propane ($C_3H_8$), and butane ($C_4H_{10}$) in different proportions, were used. The heating values $H_{HC}$ of the respective gases under the conditions of a pressure of 7 MPa and a temperature of 20°C were calculated by the calculation according to ISO6976. The compression factors $Z_{HC}$ of the respective gases under the conditions of a pressure of 7 MPa and a temperature of 20°C were also calculated by the calculation method according to ISO12213-3. As a result, in all the cases, the correlation between the heating value $H_{HC}$ and the compression factor $Z_{HC}$ approximately coincide (respective curves indicating the relations of the three types of gases for the most part overlap in Fig. 6). Therefore, these correlations can be expressed by a polynomial formula of the heating value $H_{HC}$, as shown in the formula (2) below. [Mathematical formula 2]

$$Z_{HC}(7MPa, 20°C)$$
$$= -1.073785 \times 10^{-5} H_{HC}^3 + 0.001165474 H_{HC}^2 - 0.05154084 \times H_{HC} + 1.763694 \qquad (2)$$

Here, the applicant of the present invention examined the relation between the heating value $H_{HC}$ and the compression factor $Z_{HC}$ as a relation independent of the pressure P and the temperature T. Then, for two types of gases A and B, which had the correlation shown in Fig. 6 and which were different in heating value (for example, the heating value of gas A < the heating value of gas B), normalization of the compression factor was performed using the formula (3) so that a solution to a compression factor $Z_A(T,P)$ of the gas A and a solution to a compression factor $Z_B(T,P)$ of the gas B were equal to 0 and -1, respectively.

[Mathematical formula 3]

$$N_A^B = -\frac{Z_{HC}(T,P) - Z_A(T,P)}{Z_B(T,P) - Z_A(T,P)} \qquad (3)$$

As an example, assuming that the gas A was a gas with a heating value $H_{HC}$ of 39.933 MJ/m$^3$ (pure methane, a gas of CH$_4$ = 100%) and the gas B was a gas with a heating value of 46.547 MJ/m$^3$ (a gas having a composition of CH$_4$: C$_2$H$_6$: C$_3$H$_8$: C$_4$H$_{10}$ = 86: 8: 4: 2), the compression factors of the respective gases were normalized using the formula (3) so that the solution to a compression factor $Z_{39.933MJ}(T,P)$ of the gas A and the solution to a compression factor $Z_{46.547MJ}(T,P)$ of the gas B were equal to 0 and -1, respectively.

As a result, it was found that the normalized value (N) can be expressed approximately by the function of only the heating value $H_{HC}$ as shown in the following formula (4-1).

[Mathematical formula 4]

$$N_{39.933MJ}^{46.547MJ} = -\frac{Z_{HC}(T,P) - Z_{39.933MJ}(T,P)}{Z_{46.547MJ}(T,P) - Z_{39.933MJ}(T,P)} \tag{4}$$

$$\approx -0.000144197 \times H_{HC}^3 + 0.0156245 \times H_{HC}^2 - 0.690859 \times H_{HC} + 11.8568 \tag{4-1}$$

Thus, when a natural gas was composed only of paraffin-based hydrocarbon gases, it was possible to express the result (N) of normalizing the compression factor Z, as a polynomial formula of the heating value $H_{HC}$ independent of the pressure P and the temperature T. The result (N) is hereinafter referred to as the "normalized compression factor N". The normalized compression factor N in the formula has suffixes corresponding to the respective heating values of the gas A and the gas B (an upper suffix: 46.547MJ, and a lower suffix: 39.933MJ) (N^{46.547MJ}_{39.933MJ}). In the following descriptions, these suffixes are omitted, and the normalized compression factor is simply expressed as "N".

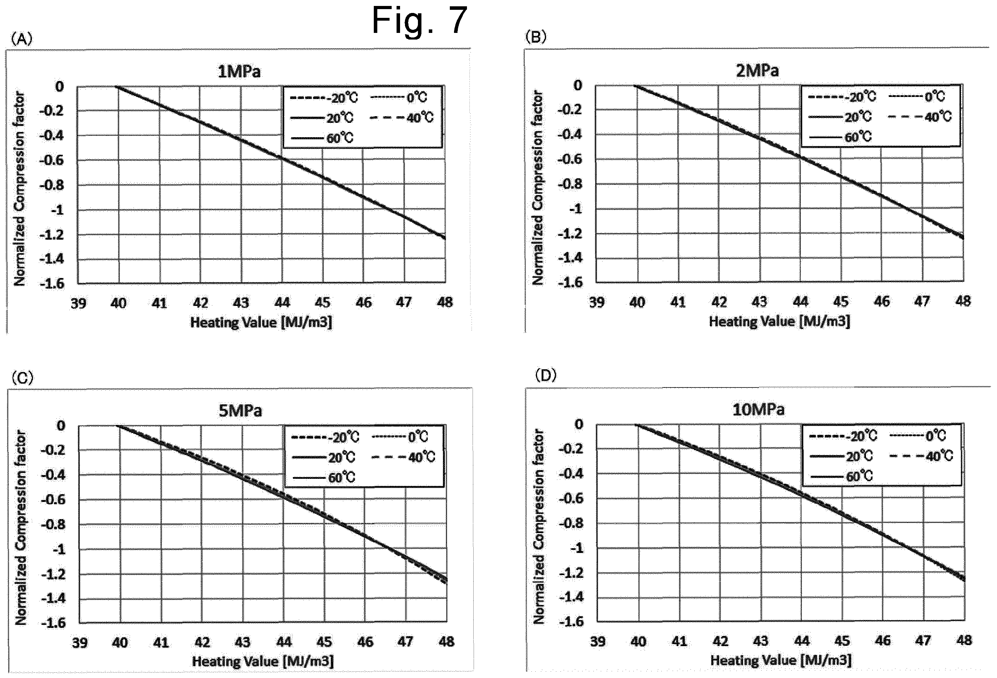

Fig. 7 shows the results of verification of the normalized compression factors N calculated by the formula (4), in the form of graphs indicating the relation between a measured value of the heating value $H_{HC}$ and the normalized compression factor N under conditions of different pressures P and temperatures T. Herein, Fig. 7(A) shows the case of the pressure being 1 MPa, Fig. 7(B) shows the case of the pressure being 2 Mpa, Fig. 7(C) shows the case of the pressure being 5 Mpa, and Fig. 7(D) shows the case of the pressure being 10 Mpa. In each case, the heating values $H_{HC}$ of the natural gas (gas composed only of paraffin-based hydrocarbon gases) under the conditions of temperatures of -20°C, 0°C, 20°C, 40°C, and 60°C were obtained by calculations according to ISO6976. The normalized compression factors N under the above-described conditions were also calculated using the formula (4). The horizontal axis represents the heating value $H_{HC}$, and the vertical axis represents the normalized compression factor N. It was found out that curves indicating the correlation between the heating value $H_{HC}$ and the normalized compression factor N can be expressed for the most part by a single curve even when the pressure P and the temperature T varies between any of the conditions shown in Figs. 7. On the basis of this result, it was also found that when a natural gas is composed only of paraffin-based hydrocarbon gases, the normalized compression factor N can be expressed approximately by the polynomial formula (4-1) of the heating value $H_{HC}$.

Next, examination was made regarding the influence of the gas, composed only of paraffin-based hydrocarbon gases having the heating value $H_{HC}$ and the normalized compression factor $N_{HC}$, on the normalized compression factor $N_{HC}$, when a nitrogen gas $x_{N2}$ [mol%] and a carbon dioxide gas $x_{CO2}$ [mol%] were added.

As a result, it was found that the normalized compression factor $N(x_{N2}, x_{CO2})$ of a natural gas, when a nitrogen gas $x_{N2}$ [mol%] and a carbon dioxide gas $x_{CO2}$ [mol%] are added, can also be expressed approximately by the function of the heating values $H_{HC}$ of paraffin-based hydrocarbon gas components, without receiving significant influence of the pressure P and the temperature T, as shown by the formula (5) below.

[Mathematical formula 5]

$$N_{39.933MJ}^{46.547MJ}(x_{N2}, x_{CO2}) = N_{HC} + k_{N2}x_{N2} + k_{CO2}x_{CO2} \tag{5}$$

provided that

$$N_{HC} = -0.000144197 \times H_{HC}^3 + 0.0156245 \times H_{HC}^2 - 0.690859 \times H_{HC} + 11.8568 \tag{5.1}$$

$$k_{N2} = a_{N2} \times H_{HC}^2 - b_{N2} \times H_{HC} + c_{N2} \tag{5.2}$$

$a_{N2}$ = 0.000139157, $b_{N2}$ = 0.00972504, $c_{N2}$ = 0.189587 , here

$$k_{CO2} = 0.0000535291 \times H_{HC}^2 - 0.00414912 \times H_{HC} + 0.0588733 \qquad (5.3)$$

A coefficient $k_{N2}$ is a coefficient ($N_2$ correction coefficient) that corrects a change caused by a nitrogen gas contained as miscellaneous gas components in a natural gas (influence of a nitrogen gas as miscellaneous gas), and $k_{CO2}$ is a coefficient ($CO_2$ correction coefficient) that corrects a change caused by a carbon dioxide gas contained as miscellaneous gas components in a natural gas (influence of a carbon dioxide gas as a miscellaneous gas). The coefficients $k_{N2}$ and $k_{CO2}$ can also be expressed approximately by a polynomial formula of the heating value $H_{HC}$ of the paraffin-based hydrocarbon gas components in a natural gas.

As repeatedly stated, the heating value $H_{HC}$ described so far represents the heating value when a natural gas is composed only of paraffin-based hydrocarbon gases or represents the heating value of only the paraffin-based hydrocarbon gas components in a natural gas (i.e., the basic heating value $H_{HC}$ of the natural gas) excluding the miscellaneous gas components when the natural gas contains miscellaneous gases (a nitrogen gas and a carbon dioxide gas).

Here, when a natural gas contains a nitrogen gas (a nitrogen gas concentration $x_{N2}$ [mol%]) and a carbon dioxide gas (a carbon dioxide gas concentration $x_{CO2}$ [mol%]) as miscellaneous gas components, the relation between the gross heating value Hs and the basic heating value $H_{HC}$ of the natural gas is expressed by the formula (6) below.
[Mathematical formula 6]

$$H_{HC} = H_S \times \frac{100}{100 - (x_{N2} + x_{CO2})} \qquad (6)$$

Specifically, by the formula (6), the formula (5) functions as the formula (normalized compression factor calculation formula) that calculates the normalized compression factor N, which is constituted of three input parameters, i.e., the heating value Hs of a natural gas, the carbon dioxide gas concentration $x_{CO2}$, and the nitrogen gas concentration $x_{N2}$ and which is not influenced by the pressure P or the temperature T. Therefore, as for the natural gas containing miscellaneous gas components, it can be said that when the heating value (gross heating value) Hs of the natural gas, the carbon dioxide gas concentration $x_{CO2}$, and the nitrogen gas concentration $x_{N2}$ can be acquired, the basic heating value $H_{HC}$ can be calculated by the formula (6), and the normalized compression factor N of the natural gas can be calculated by the formula (5).

On the basis of this consideration, in the normalized compression factor calculation method described with reference to Fig. 4(A) described above, the normalized compression factor N is calculated with three elements, i.e., the heating value (gross heating value) Hs of a target gas (a natural gas), the carbon dioxide gas concentration $x_{CO2}$, and the nitrogen gas concentration $x_{N2}$ as input parameters, using the above-described formula (5) (and the formula (6)) as the normalized compression factor calculation formula. By introducing the concept of the normalized compression factor N, the compression factor can be calculated by converting the normalized compression factor N into a general formula for calculation of the compression factor Z and then by optionally changing only the parameters, the pressure P and the temperature T.

<Calculation method of compression factor using opt-sonic arithmetic operation>

In the normalized compression factor calculation method shown in Fig. 4(A), the carbon dioxide gas concentration $x_{CO2}$ and the nitrogen gas concentration $x_{N2}$, which are miscellaneous gas components, (i.e., the measurements of these) are essential as input parameters. The applicant of the present invention further examined the method of calculating the compression factor Z using the Opt-Sonic arithmetic operation without measuring the individual concentrations of miscellaneous gas components.

As described before, the Opt-Sonic arithmetic operation can calculate the heating value Hs on the basis of, for example, the refractive index-converted heating value $H_{OPT}$ obtained from a refractive index of a target gas measured by an optical sensor or the like and on the basis of, for example, the density-converted heating value $H_{SONIC}$ obtained from a density of a target gas measured by a sound speed sensor or the like, and by using an arithmetic formula of a converted heating value (Opt-Sonic arithmetic formula) shown by the formula (7) below. In other words, the arithmetic formula of a converted heating value (Opt-Sonic arithmetic formula) described with reference to Figs. 1 to 5 described above is expressed by the formula (7) below.
[Mathematical formula 7]

$$Hs = H_{OPT} - \frac{H_{OPT} - H_{SONIC}}{1 - \alpha} \qquad (7)$$

Here, a second term of the right side of the formula (7) indicates the value of an error component, due to miscellaneous gas contained in the natural gas, included in the refractive index-converted heating value $H_{OPT}$. The value corresponds to the "error correction value" in the description with reference to Figs. 1 to 5 described above. A variable "$\alpha$" in a second term of the right side is a correction coefficient that may be substantially constant regardless of the types of miscellaneous gases when considered within a certain range of values (a range of certain gas types). As an example, the value of the correction coefficient $\alpha$ is in the range of $\alpha$ = 1.5 to 3.5.

As a result of the consideration by the applicant of the present invention, it was found that when the miscellaneous gas in the natural gas can be regarded as being composed only of a nitrogen gas and a carbon dioxide gas, a second term of the right side is proportional to the sum of the value of the nitrogen gas concentration $x_{N2}$ and the value of the carbon dioxide gas concentration $x_{CO2}$ multiplied by 1.56 as shown in the formula (8) below. Note that even if components other than a nitrogen gas and a carbon dioxide gas (for example, a hydrogen gas, a carbon monoxide gas, etc.) are contained as miscellaneous gas components, the proportional content of components other than a nitrogen gas and a carbon dioxide gas in a natural gas is very small, and there seems to be no influence on the calculation values in this consideration (this also applies to the following).

[Mathematical formula 8]

$$\frac{1}{0.2529} \times \frac{H_{OPT} - H_{SONIC}}{1 - \alpha} = (x_{N2} + 1.56 \times x_{CO2}) = y \qquad (8)$$

Accordingly, by regarding the result of the formula (8) as a variable y, and manipulating the variable y and assigning it to the right side of the formula (6), a value corresponding to the basic heating value $H_{HC}$ in the formula (6) can be calculated (the formula (9) below). The variable y is a total approximate concentration of a nitrogen gas and a carbon dioxide gas, i.e., the "miscellaneous gas total approximate concentration y" in the descriptions referring to Figs. 1 to 5 described above, and the formula (8) is a miscellaneous gas total approximate concentration calculation formula. As shown by the formula (8), the "miscellaneous gas total approximate concentration y" can be calculated from the Opt-Sonic arithmetic operation. In other words, even when the respective concentrations of miscellaneous gas components (the nitrogen gas concentration $x_{N2}$ and the carbon dioxide gas concentration $x_{CO2}$) are unknown (that is, without measuring the concentrations of the miscellaneous gas components), the miscellaneous gas total approximate concentration y can be calculated on the basis of the refractive index-converted heating value $H_{OPT}$ and the density-converted heating value $H_{SONIC}$. For the formula (6), when the carbon dioxide gas concentration $x_{CO2}$ that is approximated to be 1.56 times the actual value is used instead of the carbon dioxide gas concentration $x_{CO2}$, and the nitrogen gas concentration $x_{N2}$ and the carbon dioxide gas concentration $x_{CO2}$ are forcibly replaced with the miscellaneous gas total approximate concentration y by allowing an approximation error, the following formula (9) is obtained.

[Mathematical formula 9]

$$H'_{HC} = Hs \times \frac{100}{100 - y} = Hs \times \frac{100}{100 - (x_{N2} + 1.56 \times x_{CO2})} \qquad (9)$$

As a result, for the formula (9), the variable Hs can be calculated from the Opt-Sonic arithmetic operation in the formula (7), and the variable y can be calculated from the Opt-Sonic arithmetic operation in the formula (8). Therefore, the formula (9) is a formula that uses the Opt-Sonic arithmetic operation to virtually calculate the basic heating value (the virtual basic heating value $H'_{HC}$). The formula (9) is the "virtual basic heating value calculation formula" in the descriptions referring to Figs. 1 to 5 described above. The virtual basic heating value $H'_{HC}$ can be used as a substitute value (approximate value) for the basic heating value $H_{HC}$ of a natural gas obtained by measuring the respective concentrations of miscellaneous gas components and performing calculation by the formula (6).

Then, in the calculation formula of the normalized compression factor N by the formula (5), when the basic heating value $H_{HC}$ is replaced with the virtual basic heating value $H'_{HC}$ of the above-described formula (9) with minor modifications, the formula (10) below can be acquired. Therefore, the formula (10) is also a normalized compression factor calculation formula similar to the formula (5). As described before, since the virtual basic heating value $H'_{HC}$ can be calculated using the Opt-Sonic arithmetic operation, the normalized compression factor of the formula (10) can be calculated even when the concentrations of miscellaneous gas components (the nitrogen gas concentration $x_{N2}$ and the carbon dioxide gas concentration $x_{CO2}$) are unknown.

[Mathematical formula 10]

$$N_{39.933MJ}^{46.547MJ} = N'_{HC} + k' \times y = N'_{HC} + k'x_{N2} + 1.56 \times k'x_{CO2} \qquad (10)$$

provided that

$$N'_{HC} = -0.000144197 \times H'^{3}_{HC} + 0.0156245 \times H'^{2}_{HC} - 0.690859 \times H'_{HC} + 11.8568 \quad (10.1)$$

$$k' = 0.85 \times a_{N2} \times H'^{2}_{HC} - 0.85 \times b_{N2} \times H'_{HC} + 0.85 \times c_{N2} \quad (10.2)$$

, here $a_{N2} = 0.000139157$, $b_{N2} = 0.00972504$, $c_{N2} = 0.189587$

In the formulas (10) and (10.1), $N'_{HC}$ is a virtual normalized compression factor that does not include (excludes) the miscellaneous gas components and that corresponds to the virtual basic heating value $H'_{HC}$ not including (excluding) the miscellaneous gas components. In other words, $N'_{HC}$ is an arithmetic normalized compression factor, that is, a virtual normalized compression factor that assumes that a natural gas is composed only of paraffin-based hydrocarbon (without including miscellaneous gases) and that the basic heating value is a virtual basic heating value H.

In the formula (10), k' is a coefficient to correct a change in the virtual normalized compression factor, which corresponds to the virtual basic heating value $H'_{HC}$. This change is due to the natural gas containing a nitrogen gas and a carbon dioxide gas as miscellaneous gas components (an overall influence of the nitrogen gas and the carbon dioxide gas as miscellaneous gases). Hereinafter, k' is referred to as a "miscellaneous gas correction coefficient".

The formula (10.1) is the same in terms of coefficients to the formula (5.1), while the basic heating value $H_{HC}$ in the formula (5.1) is replaced with the virtual basic heating value $H'_{HC}$.

The formula (10.2) is a formula to select and calculate the miscellaneous gas correction coefficient (k'), this calculation being made with reference to the $N_2$ correction coefficient $k_{N2}$ of the formula (5.2). Specifically, in order to "correct the overall influence of a nitrogen gas and a carbon dioxide gas as miscellaneous gases", a coefficient ($N_2$ correction coefficient $k_{N2}$) that "corrects the influence of the nitrogen gas as a miscellaneous gas" is used. As the basic heating value, the virtual basic heating value $H'_{HC}$ is used. Specifically, the basic heating value $H_{HC}$ of the right side of the formula (5.2) is replaced with the virtual basic heating value $H'_{HC}$, and coefficients $a_{N2}$, $b_{N2}$, and $c_{N2}$ in respective terms of the right side of the formula (5.2) are multiplied by a prescribed constant (0.85 in this case) to calculate the miscellaneous gas correction coefficient k'. The prescribed constant (0.85) in this case is a value that can be set (adjusted) on the basis of the type (the concentration of a carbon dioxide gas that is assumed to be contained in the target gas as a miscellaneous gas) of a target gas (a natural gas). The prescribed constant is hereinafter referred to as an "adjustment coefficient D". The adjustment coefficient D will be described later in detail.

Therefore, in the formula (10), as a change (amount) due to the presence of miscellaneous gas components, the miscellaneous gas total approximate concentration y is used instead of the values (measured values) of the nitrogen gas concentration $x_{N2}$ and the carbon dioxide gas concentration $x_{CO2}$. A change (deviation) caused by using the miscellaneous gas total approximate concentration y is corrected by the miscellaneous gas correction coefficient k' calculated on the basis of the $N_2$ correction coefficient $k_{N2}$.

Thus, according to the formula (10), the normalized compression factor N, which is not influenced by the pressure P or the temperature T, can be calculated on the basis of two parameters, the refractive index (refractive index-converted heating value $H_{OPT}$) and the density (density-converted heating value $H_{SONIC}$) of a natural gas.

On the basis of this consideration, in the normalized compression factor calculation method described with reference to Fig. 4(B) described above, two elements of the refractive index and the density of a natural gas are used as input parameters, and the above-described formula (10) is used as the normalized compression factor calculation formula to calculate the normalized compression factor N. By introducing the concept of the normalized compression factor N, the compression factor can be calculated by converting the normalized compression factor into a general formula for calculation of the compression factor and then by optionally changing only the parameters, the pressure P and the temperature T. In addition, the number of the input parameters can be reduced as compared to the normalized compression factor calculation method using the formula (5).

These findings are based on the fact that the normalized compression factor N by the formula (5) and the normalized compression factor N by the formula (10) produce substantially equal values for the same type of natural gases under the same conditions (pressure, temperature, etc.). This will be described below.

In the formula (5), a normalized compression factor $N_{HC}$ is calculated on the basis of the basic heating value $H_{HC}$ of a natural gas, and the correction terms corresponding to individually measured nitrogen gas concentration $x_{N2}$ and carbon dioxide gas concentration $x_{CO2}$ are added to the normalized compression factor $N_{HC}$ to correct an error due to the miscellaneous gas components, so as to calculate the normalized compression factor $N(x_{N2}, x_{CO2})$ in consideration of the miscellaneous gas components. Specifically, the nitrogen gas concentration $x_{N2}$ is multiplied by the $N_2$ correction coefficient $k_{N2}$, the carbon dioxide gas concentration $x_{CO2}$ is multiplied by the $CO_2$ correction coefficient $k_{CO2}$, and these correction terms are added.

Meanwhile, in the formula (10), the correction term of multiplying the miscellaneous gas total approximate concentration y by the miscellaneous gas correction coefficient k' is added to the arithmetic normalized compression factor, that is, a virtual normalized compression factor $N'_{HC}$ to correct an error due to the miscellaneous gas components, so as to calculate the normalized compression factor N in consideration of the miscellaneous gas components.

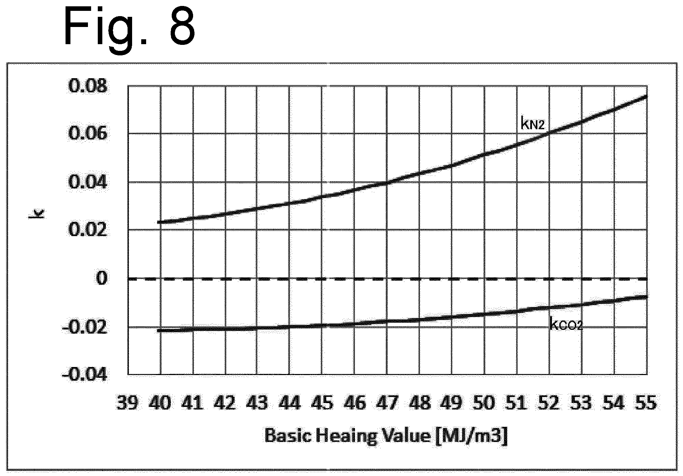

Here, attention is paid to the $N_2$ correction coefficient $k_{N2}$, the $CO_2$ correction coefficient $k_{CO2}$, and the miscellaneous gas correction coefficient k'. Considering an existing natural gas, the basic heating value thereof may be considered to be between 39.933 and 47 MJ/m$^3$ (around 55 MJ/m$^3$ at the highest). The applicant of the present invention examined possible values of the $N_2$ correction coefficient $k_{N2}$ in the formula (5.2) and the $CO_2$ correction coefficient $k_{CO2}$ in the formula (5.3) in this range of the basic heating value $H_{HC}$. The result thereof is shown in Fig. 8. In Fig. 8, the horizontal axis represents the basic heating value $H_{HC}$, and the vertical axis represents the correction coefficients $k_{N2}$ and $k_{CO2}$.

As a result, the $N_2$ correction coefficient $k_{N2}$ takes positive values and the $CO_2$ correction coefficient $k_{CO2}$ takes negative values in the range of the above-described basic heating value. This means that the normalized compression factor $N(x_{N2}, x_{CO2})$ becomes larger as the nitrogen gas concentration $x_{N2}$ increases, and that the normalized compression factor $N(x_{N2}, x_{CO2})$ becomes smaller as the carbon dioxide gas concentration $x_{CO2}$ increases.

Meanwhile, since the miscellaneous gas correction coefficient k' in the formula (10) is a value selected with reference to the $N_2$ correction coefficient $k_{N2}$ of the formula (5.2), the miscellaneous gas correction coefficient k' constantly takes positive values. This means that the term (k' $\times$ $x_{N2}$) and the term (1.56k' $\times$ $x_{CC2}$) in the formula (10) both take positive values, and therefore the miscellaneous gas correction coefficient k' of the formula (10) cannot cause the normalized compression factor N to be smaller (to be negative) when the carbon dioxide gas concentration $x_{CO2}$ increases, unlike the $CO_2$ correction coefficient $k_{CO2}$.

However, the virtual basic heating value $H'_{HC}$ not including (excluding) miscellaneous gas components is originally calculated reversely from the miscellaneous gas total approximate concentration y on the basis of the natural gas heating value (gross heating value) Hs obtained from the Opt-Sonic arithmetic operation by using the formula (9). Accordingly, when the carbon dioxide gas concentration $x_{CO2}$ is 0 mol%, the miscellaneous gas total approximate concentration y totally coincides with the nitrogen gas concentration $x_{N2}$, so that the virtual basic heating value $H'_{HC}$ becomes equal to the basic heating value $H_{HC}$ of the formula (6). However, when the carbon dioxide gas concentration $x_{CO2}$ is larger than 0 mol%, the carbon dioxide gas concentration $x_{CO2}$ included in the miscellaneous gas total approximate concentration y used in the reverse calculation is increased by 1.56 times. Accordingly, the virtual basic heating value $H'_{HC}$ has the tendency of shifting in the direction of becoming larger than the basic heating value $H_{HC}$ as the carbon dioxide gas concentration $x_{CO2}$ increases. As is clear from Fig. 7, the normalized compression factor N becomes lower as the basic heating value $H_{HC}$ is higher. Accordingly, a virtual normalized compression factor $N'_{HC}$ of the formula (10.1) corresponding to the first term of the formula (10) becomes smaller than the normalized compression factor $N_{HC}$ of the formula (5.1) corresponding to first term of the formula (5).

Meanwhile, as for the miscellaneous gas total approximate concentration y used in the second term of the formula (10), the carbon dioxide gas concentration $x_{CO2}$ included therein is made 1.56 times larger than an actual value. At the same time, the coefficient k' used in the second term includes the virtual basic heating value $H'_{HC}$ in the first term and the second term as shown in the formula (10.2). Accordingly, the virtual basic heating value $H'_{HC}$ has the property of shifting in the direction of becoming larger than the basic heating value $H_{HC}$ as the carbon dioxide gas concentration $x_{CO2}$ is larger as described above, and therefore when a carbon dioxide gas is included, k' $\times$ y in the second term of the formula (10) becomes larger than the sum of the second term and the third term of the formula (5).

Therefore, when the formula (5) and the formula (10) are compared as a whole, the first term of the formula (10) becomes smaller than the first term of the formula (5), whereas k' $\times$ y in the second term of the formula (10) becomes larger than the sum of the second term and the third term of the formula (5). As a result, it was expected that the formula (10) has the tendency of approaching (not significantly deviating from) the calculation result of the formula (5) due to an offset of the increase in the first term and the decrease in the second term. Therefore, it was considered that appropriately adjusting the first term and the second term of the formula (10) (offsetting the values thereof) can produce the miscellaneous gas correction coefficient k', which can cause the virtual normalized compression factor $N'_{HC}$ to be smaller (to be negative) when the carbon dioxide gas concentration $x_{CO2}$ increases. Hence, in the formula (10.2), it was examined to use an adjustment coefficient D (by multiplying coefficients $a_{N2}$, $b_{N2}$, and $c_{N2}$ of the respective right terms of the formula (5.2) by the adjustment coefficient D) so as to reduce an error between the result of arithmetic operation by the formula (5) and that by the formula (10) by appropriately selecting the adjustment coefficient D.

The applicant of the present invention performed a simulation to compare the compression factor calculated using ISO12213-3 and the compression factor calculated on the basis of the normalized compression factor N which is calculated (by the formula (10)) using the Opt-Sonic arithmetic operation, for natural gases having various compositions of a carbon dioxide and a nitrogen gas.

As a result, a value (for example, 0.85) that can suppress an error from the compression factor calculated using ISO12213-3 to be small was selected as the adjustment coefficient D of the formula (10.2) for both the natural gas with a high amount of a carbon dioxide gas and the natural gas with a high amount of a nitrogen gas.

Although the adjustment coefficient D is 0.85 in the above-described example, the adjustment coefficient D is not limited thereto, and appropriate values can be selected in accordance with the types of natural gases. For example, in the case of a gas that does not contain a carbon dioxide gas, such as an LNG vaporized gas, the adjustment coefficient D is desirably 1.0 (or values close to 1.0). In the case of a natural gas with the adjustment coefficient D = 1, i.e., a gas not containing a carbon dioxide gas, the miscellaneous gas correction coefficient k' is equal to the $N_2$ correction coefficient $k_{N2}$, and the virtual basic heating value $H'_{HC}$ is equal to the basic heating value $H_{HC}$ in the formula (6). In the case of adding a biogas to a natural gas, the influence of the carbon dioxide gas concentration can be dominant, and therefore it is desirable to set the adjustment coefficient D to be low (for example, about 0.7). The adjustment coefficient D in the present embodiment is, for example, a value of $0.5 \leq D \leq 1.5$.

Thus, according to the calculation device and the calculation method of the present embodiment, it is possible to calculate the virtual basic heating value $H'_{HC}$ using the Opt-Sonic arithmetic operation and to calculate physical quantities (for example, the compression factor) relating to the state of a natural gas by using the virtual basic heating value $H'_{HC}$ as a substitute of the basic heating value $H_{HC}$. In that case, it is possible to calculate the miscellaneous gas correction coefficient k' that can cause the virtual normalized compression factor $N'_{HC}$ in the first term of the formula (10) to be smaller (to be negative) (by an appropriate offset of the first term and the second term of the formula (10)) in accordance with increase of the carbon dioxide gas concentration $x_{CO2}$. In the calculation of the miscellaneous gas correction coefficient k', using the adjustment coefficient D makes it possible to adjust the degree of offset between the first term and the second term of the formula (10) in accordance with the increase of the carbon dioxide gas concentration $x_{CO2}$. In addition, the adjustment coefficient D can be set and adjusted to an appropriate value (0.85 in this case). For example, the calculation device 1 of the present embodiment can hold the adjustment coefficient D as a preset value (for example, an initial value), or can receive an input of any adjustment coefficient D from the outside. In the program of the present embodiment, the adjustment coefficient D may be held as a preset value (a constant or an initial value), or the adjustment coefficient D may be one of the input parameters.

As a result, both the normalized compression factor N $(x_{N2}, x_{CO2})$ calculated by the formula (5) and the normalized compression factor N calculated by the formula (10) can produce equal values for the same type of natural gases under the same conditions.

<Generalization of normalized compression factor>

In the case of converting the normalized compression factor N $(x_{N2}, x_{CO2})$ obtained by the formula (5) or the normalized compression factor N obtained by the formula (10) to a general formula Z(T, P) for the compression factor Z based on the pressure P and the temperature T, a conversion formula shown in the formula (11) below is used.
[Mathematical formula 11]

$$Z(T,P) = \boxed{-N_{39.933MJ}^{46.547MJ}(x_{N2}, x_{CO2})} \times \left( Z_{46.547MJ}(T,P) - Z_{39.933MJ}(T,P) \right) + Z_{39.933MJ}(T,P) \qquad (11)$$

provided that

$$Z_{39.933MJ}(T,P) = 1 + \sum_{m=1}^{6} \sum_{n=0}^{4} a_{m,n} T^n P^m \qquad (11.1)$$

$$Z_{46.547MJ}(T,P) = 1 + \sum_{m=1}^{6} \sum_{n=0}^{4} b_{m,n} T^n P^m \qquad (11.2)$$

Note that $a_{m,n}$, and $b_{m,n}$ included in the formula (11.1) and the formula (11.2) are coefficients determined by the heating values of gases A and B selected when the compression factor is normalized. In the present embodiment, the coefficients correspond to the gas A of 39.933 MJ/m$^3$ (CH4 = 100%) and the gas B of 46.547 MJ/m$^3$ (CH4: C2H6: C3H8: C4H10 = 86: 8: 4: 2). In this case, the coefficient $a_{m,n}$ is a value shown in Table 1 below, and the coefficient $b_{m,n}$ is a value shown in Table 2 below.

[Table 1]

$a_{m,n}$

| m＼n | 0 | 1 | 2 | 3 | 4 |
|---|---|---|---|---|---|
| 1 | $-2.35036 \times 10^{-2}$ | $2.87327 \times 10^{-4}$ | $-1.64352 \times 10^{-6}$ | $3.42170 \times 10^{-9}$ | $6.25526 \times 10^{-12}$ |
| 2 | $-5.32383 \times 10^{-5}$ | $1.05061 \times 10^{-5}$ | $-2.89761 \times 10^{-7}$ | $5.07148 \times 10^{-9}$ | $-3.64105 \times 10^{-11}$ |
| 3 | $1.96841 \times 10^{-5}$ | $-1.22527 \times 10^{-6}$ | $7.20498 \times 10^{-8}$ | $-1.77551 \times 10^{-9}$ | $1.42934 \times 10^{-11}$ |
| 4 | $-1.39998 \times 10^{-6}$ | $2.74477 \times 10^{-7}$ | $-1.66331 \times 10^{-8}$ | $3.93329 \times 10^{-10}$ | $-3.09583 \times 10^{-12}$ |
| 5 | $2.92517 \times 10^{-7}$ | $-3.10250 \times 10^{-8}$ | $1.57764 \times 10^{-9}$ | $-3.54783 \times 10^{-11}$ | $2.74550 \times 10^{-13}$ |
| 6 | $-1.03879 \times 10^{-8}$ | $9.51030 \times 10^{-10}$ | $-4.63848 \times 10^{-11}$ | $1.03507 \times 10^{-12}$ | $-8.00392 \times 10^{-15}$ |

[Table 2]

$b_{m,n}$

| m＼n | 0 | 1 | 2 | 3 | 4 |
|---|---|---|---|---|---|
| 1 | $-3.45532 \times 10^{-2}$ | $3.53753 \times 10^{-4}$ | $-2.12257 \times 10^{-7}$ | $-2.96449 \times 10^{-8}$ | $2.18618 \times 10^{-10}$ |
| 2 | $-1.12462 \times 10^{-3}$ | $9.73014 \times 10^{-5}$ | $-3.79388 \times 10^{-6}$ | $7.08069 \times 10^{-8}$ | $-4.83187 \times 10^{-10}$ |
| 3 | $3.10569 \times 10^{-4}$ | $-3.66076 \times 10^{-5}$ | $1.82219 \times 10^{-6}$ | $-3.82960 \times 10^{-8}$ | $2.79044 \times 10^{-10}$ |
| 4 | $-6.34425 \times 10^{-5}$ | $8.04882 \times 10^{-6}$ | $-4.18906 \times 10^{-7}$ | $9.12908 \times 10^{-9}$ | $-6.83021 \times 10^{-11}$ |
| 5 | $6.13475 \times 10^{-6}$ | $-7.50572 \times 10^{-7}$ | $4.01000 \times 10^{-8}$ | $-8.96848 \times 10^{-10}$ | $6.83158 \times 10^{-12}$ |
| 6 | $-1.86118 \times 10^{-7}$ | $2.34659 \times 10^{-8}$ | $-1.30916 \times 10^{-9}$ | $3.00693 \times 10^{-11}$ | $-2.32617 \times 10^{-13}$ |

The present embodiment is described in the case where the gas A is a gas (pure methane, a gas of $CH_4$ = 100%) with a heating value $H_{HC}$ of 39.933 MJ/m³, and the gas B is a gas with a heating value $H_{HC}$ of 46.547 MJ/m³ (a gas having a composition of $CH_4$: $C_2H_6$: $C_3H_8$: $C_4H_{10}$ = 86: 8: 4: 2). However, without being limited thereto, any gases, such as gases available at the site can be adopted as the gas A and gas B. In that case, the coefficients $a_{m,n}$ and $b_{m,n}$ are prescribed values according to the adopted gases.

The calculation result of the formula (5) or the calculation result of the formula (10), or the result of output from the normalized compression factor calculation unit 151 (see Fig. 2(B)) of the calculation device 1 in the present embodiment is used as the value of the normalized compression factor $N(x_{N2}, x_{CO2})$ in the first term (a dashed square frame) of the right side of the formula (11). The normalized compression factor N by the formula (10) can also be used as an equal value. Then, by substituting optional temperature T and pressure P into the formula (11), the natural gas compression factor Z under the conditions of the temperature T and the pressure P can be obtained.

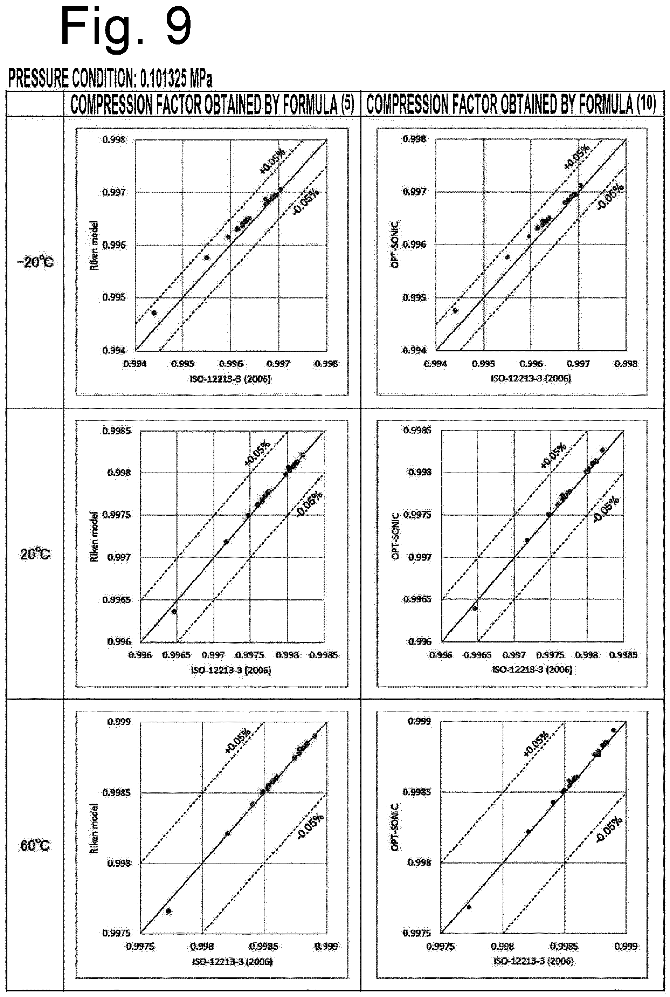

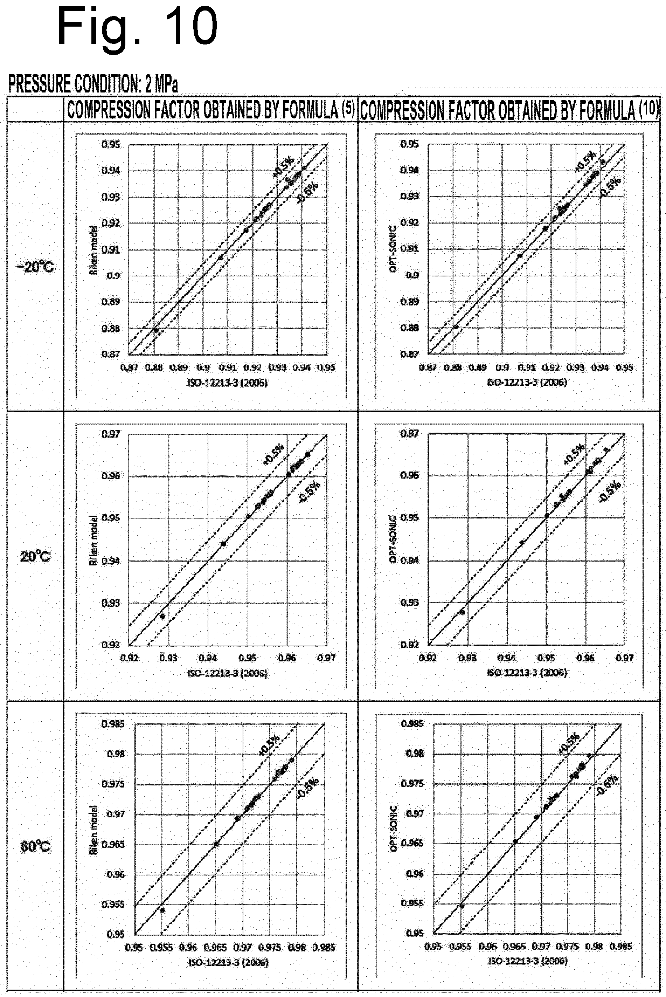

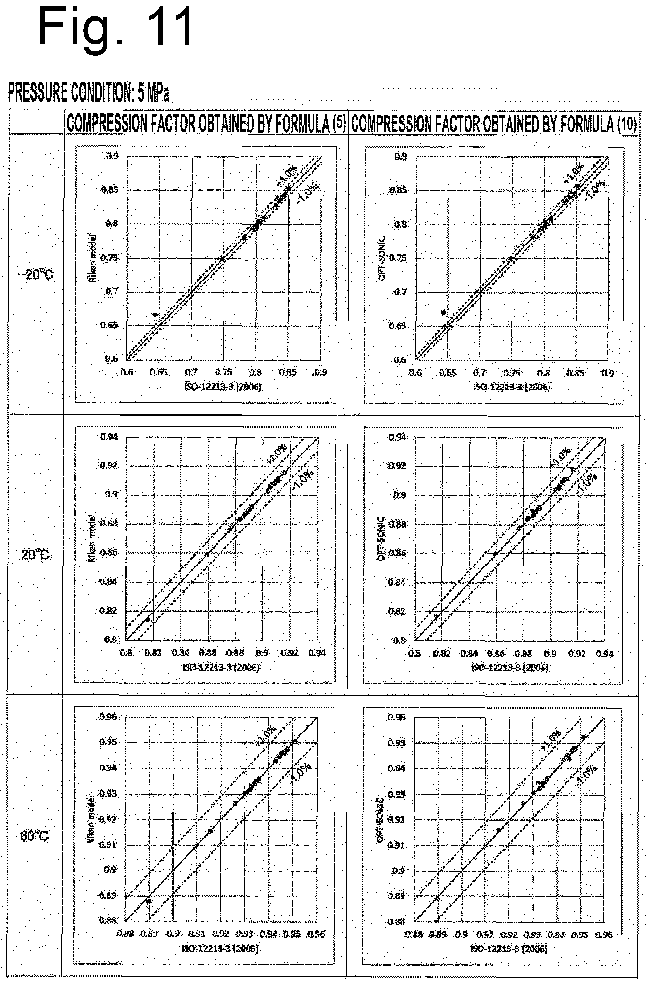

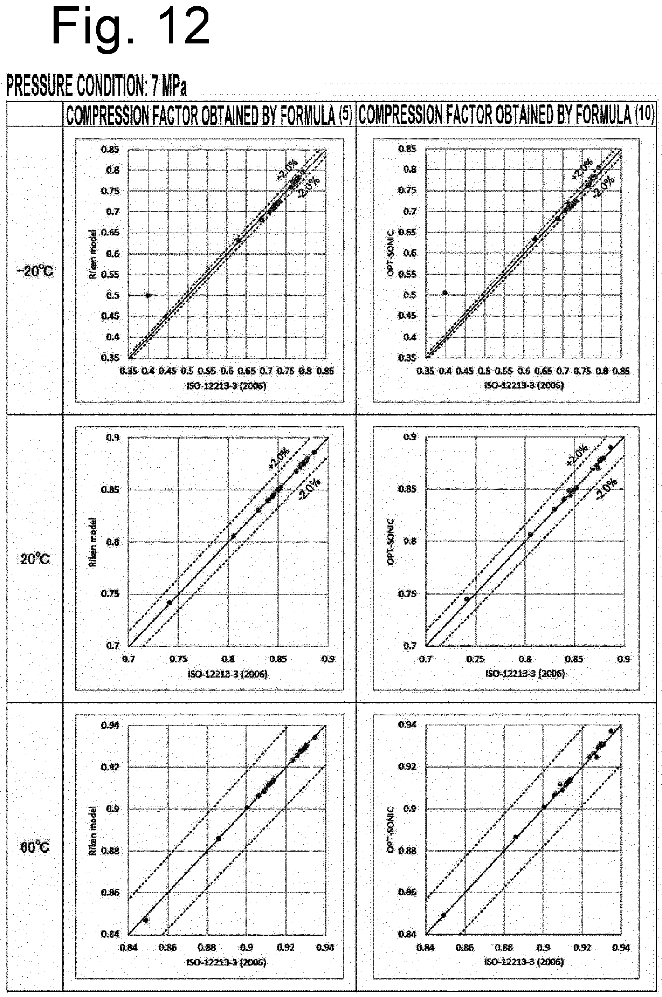

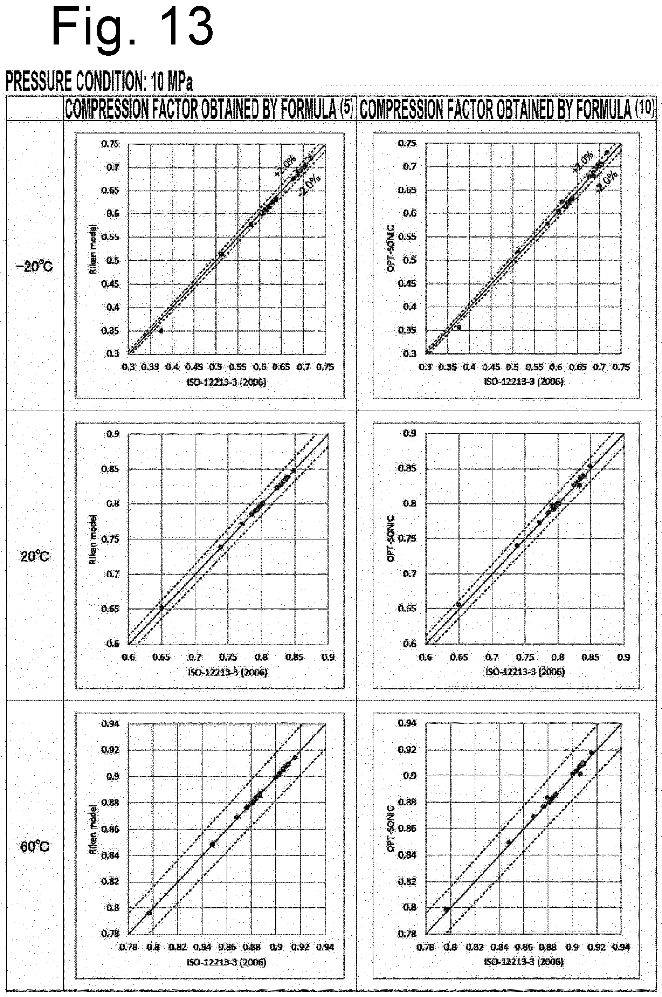

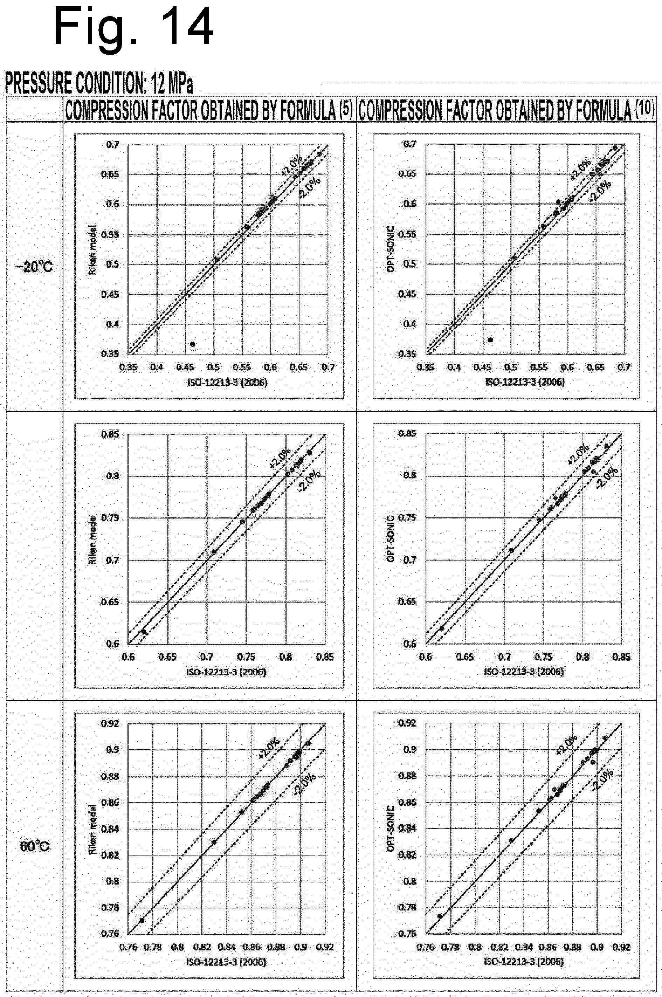

Fig. 9 shows graphs comparing a compression factor obtained by the conventional calculation method according to ISO12213-3, a compression factor obtained by the formula (11) on the basis of the normalized compression factor $N(x_{N2}, x_{CO2})$ calculated by the formula (5) of the present embodiment, and a compression factor obtained by the formula (11) on the basis of the normalized compression factor N calculated by the formula (10). The horizontal axis represents a compression factor Z obtained by the calculation method according to ISO12213-3. The vertical axis represents a compression factor Z obtained by the formula (11) on the basis of the normalized compression factor $N(x_{N2}, x_{CO2})$ calculated by the formula (5) of the present embodiment and a compression factor Z obtained by the formula (11) on the basis of the normalized compression factor N calculated by the formula (10). The pressure (P) conditions were 0.101325 MPa (Fig. 9), 2 MPa (Fig. 10), 5 MPa (Fig. 11), 7 MPa (Fig. 12), 10 MPa (Fig. 13), and 12 MPa (Fig. 14). The temperature (T) conditions of -20°C, 20°C and 40°C for each of the pressure (P) conditions were used for calculation.

As a result of the calculations, it was found that the error from the calculation result of the compression factor according to ISO12213-3 falls within the range of $\pm$ 0.05% to $\pm$ 2.0% under different pressure P and temperature T conditions, and a high degree of agreement was thus demonstrated.

Thus, according to the present embodiment, by adopting the concept of the normalized compression factor N, the compression factor of a natural gas can be calculated simply and easily as compared to the conventional methods (calculation methods according to ISO12213-2 and ISO12213-3).

When the Opt-Sonic arithmetic operation is used to calculate the virtual basic heating value $H'_{HC}$ in particular, physical property values relating to the combustion or the state of a natural gas can be calculated. In particular, in the calculation of various physical property values (for example, the compression factor) that can be calculated using the basic heating value $H_{HC}$ of a natural gas, the virtual basic heating value $H'_{HC}$ can be used. In the above-described example, when the virtual basic heating value $H'_{HC}$ is used for the compression factor Z, which can be calculated by using the basic heating value $H_{HC}$, the number of input parameters used for the calculation can be reduced. In addition to the number of input parameters being reduced, the input parameters to be used are values easily acquired, such as the refractive index and the density of a natural gas (the values acquired by devices that are small, simple, inexpensive, and easy to handle). Accordingly, as compared to the case of using, for example, conventional analytical values of gas chromatography, physical property values (for example, the compression factor) can be calculated simply and easily.

Various physical property values that can be calculated using the basic heating value $H_{HC}$ are not limited to the compression factor. For example, even combustion speeds can be adopted in a similar manner. This will be described below.

<Calculation method of combustion speed>

The applicant of the present invention verified how the compositions of a plurality of types of natural gases influence the basic heating value and the combustion speed (MCP). The natural gases used for verification are mixed gases composed only of three different types of paraffin-based hydrocarbon gases: a $(CH_4\text{-}C_2H_6)$ gas, a $(CH_4\text{-}C_3H_8)$ gas; and a $(CH_4\text{-}C_4H_{10})$ gas.

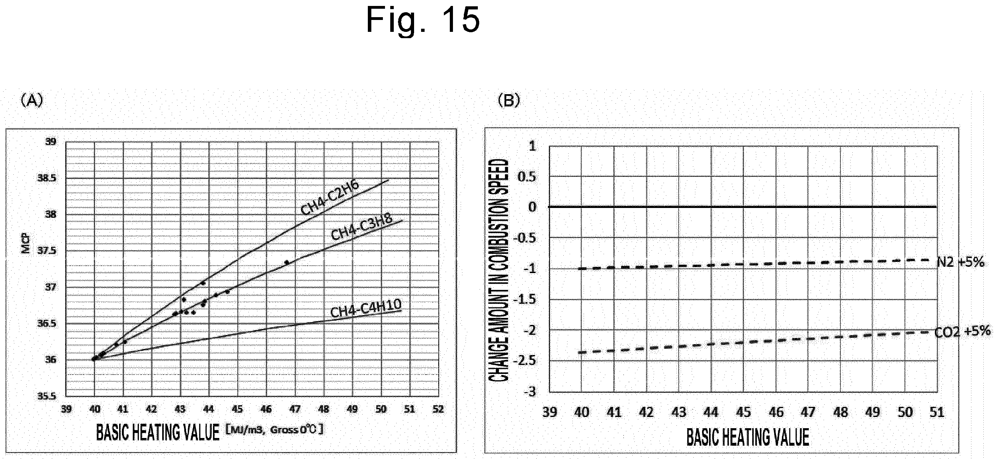

Fig. 15(A) is a graph showing the relation between the basic heating values of the three types of mixed gases (calculated values based on IS06976) and the values by a calculation formula ((Pa) formula) of the combustion speed MCP using the analytical values of gas chromatography according to the conventional methods. The horizontal axis represents the basic heating value [MJ/m$^3$], and the vertical axis represents the combustion speed MCP. As a result, it was found that the correlation between the basic heating value and the MCP can be much different depending on the composition of natural gases (mixed gases).

However, as a result of performing similar verification of the correlation using existing natural gases (natural gases having compositions listed in ISO TR22302:2014, Table B.6, No.1 to 24), the results of the relation between the basic heating value $H_{HC}$ and the combustion speed MCP showed the correlation close to that of the $(CH_4\text{-}C_3H_8)$ gas. The results indicate that the combustion speed MCP of existing natural gases can be approximately expressed by the following formula (12).

[Mathematical formula 12]

$$\text{MCP} = -0.004637 \times H_{HC}^2 + 0.5961 \times H_{HC} + 19.5965 \qquad (12)$$

A mixed gas was formed by mixing the $(CH_4\text{-}C_3H_8)$ gas as shown in Fig. 15(A), with 5 mol% nitrogen gas and 5 mol% carbon dioxide gas as miscellaneous gas components, and the relation between a change amount in combustion speed and the basic heating value of the mixed gas was verified with the $(CH_4\text{-}C_3H_8)$ gas as a reference (0). The result is shown in Fig. 15(B). The result of the verification indicates that the change amount in combustion speed due to the miscellaneous gas components being mixed therein becomes larger with an increase in the basic heating value.

The result indicates that when a natural gas (an existing natural gas), having a basic heating value in the vicinity of the curve (Fig.15(A)) of the reference $(CH_4\text{-}C_3H_8)$ gas containing no miscellaneous gas components, is mixed with $x_{N2}$ [mol%] nitrogen gas and $x_{CO2}$ [mol%] carbon dioxide gas as miscellaneous gas components, the combustion speed can be expressed by the formula (13).

[Mathematical formula 13]

$$\text{MCP} = -0.004637 \times H_{HC}^2 + 0.5961 \times H_{HC} + 19.5965 + A \times x_{N2} + B \times x_{CO2} \qquad (13)$$

provided that

$$A = 0.00265438 \times H_{HC} - 0.30474328 \qquad (13.1)$$

$$B = 0.0062857 \times H_{HC} - 072340434 \qquad (13.2)$$

Note that the heating value $H_{HC}$ used in the formula (13) represents the basic heating value of a natural gas. Here, when the natural gas contains a nitrogen gas (the nitrogen gas concentration $x_{N2}$ [mol%]) and a carbon dioxide gas (the carbon dioxide gas concentration $x_{CO2}$ [mol%]) as miscellaneous gas components, the relation between the gross heating value Hs and the basic heating value $H_{HC}$ of the natural gas is expressed by the formula (14) below.

[Mathematical formula 14]

$$H_{HC} = H_S \times \frac{100}{100 - (x_{N2} + x_{CO2})} \qquad (14)$$

Therefore, by the formula (14), the formula (13) functions as a formula for calculating the combustion speed on the basis of three input parameters, i.e., the heating value Hs of a natural gas, the carbon dioxide gas concentration $x_{CO2}$, and the nitrogen gas concentration $x_{N2}$. In other words, in the case of a natural gas containing miscellaneous gas components, it can be said that when the heating value (gross heating value) Hs of the natural gas, the carbon dioxide gas concentration $x_{CO2}$, and the nitrogen gas concentration $x_{N2}$ can be acquired, it is possible to calculate the basic heating value $H_{HC}$ by the formula (14) and to calculate the combustion speed MCP of the natural gas by the formula (13).

On the basis of this consideration, the combustion speed calculation method described with reference to Fig. 5(A) described above calculates the combustion speed MCP with three elements, i.e., the heating value (gross heating value) Hs of a natural gas, the carbon dioxide gas concentration $x_{CO2}$, and the nitrogen gas concentration $x_{N2}$ as input parameters, using the above-described formula (13) (and the formula (14)) as the normalized compression factor calculation formula.

The formula (13) (and the formula (14)) are formulas for calculating combustion speed (combustion speed calculation formulas) on the basis of three input parameters, i.e., the heating value Hs of a natural gas, the carbon dioxide molar concentration $x_{CO2}$, and the nitrogen molar concentration $x_{N2}$.

<Method of Arithmetic Operation of Opt-Sonic Combustion Speed>

Incidentally, in calculation of the combustion speed, the number of input parameters can be reduced by using the Opt-Sonic arithmetic operation, in exactly the same way as in the calculation of the compression factor. In addition, the virtual basic heating value $H'_{HC}$ can also be calculated in exactly the same way as in the calculation of the compression factor (the formulas (7) to (9) described above).

Even when the concentrations of miscellaneous gas components (the nitrogen gas concentration $x_{N2}$ and the carbon dioxide gas concentration $x_{CO2}$) are unknown, an arithmetic operation corresponding to the calculation of the combustion speed by the formula (13) is performed by the formula (15) below by substituting the virtual basic heating value $H'_{HC}$ for the basic heating value $H_{HC}$ of the formula (13). Therefore, the formula (15) is also a combustion speed calculation formula.

[Mathematical formula 15]

$$\mathrm{MCP} = -0.004637 \times H'^{2}_{HC} + 0.5961 \times H'_{HC} + 19.5965 + C \times (x_{N2} + 1.56 \times x_{CO2}) \qquad (15)$$

provided that

$$\mathrm{C} = 1.1 \times \mathrm{A} = 1.1 \times (0.00265438 \times H'_{HC} - 0.30474328) \qquad (15.1)$$

Here, when the formula (13) is compared to the formula (15), in the formula (13), a fourth term ($A \times x_{N2}$) and a fifth term ($B \times x_{CO2}$) of the right side are added as correction terms required due to the miscellaneous gas components being included. In the formula (15), a fourth term ($C \times (x_{N2} + 1.56 \times x_{CO2})$) of the right side is added as a correction term required due to the miscellaneous gas components being included. With reference to Fig. 15(B) again, in calculation of the combustion speed by the formula (13), as any of the nitrogen gas concentration $x_{N2}$ and the carbon dioxide gas concentration $x_{CO2}$ increases, the change amount in combustion speed tends to decrease. However, the influence of the carbon dioxide gas concentration $x_{CO2}$ on the change amount in combustion speed may be considered substantially twice the influence of the nitrogen gas concentration $x_{N2}$.

In the formula (15) using the Opt-Sonic arithmetic operation, the influence of the carbon dioxide gas concentration $x_{CO2}$ on the change amount in combustion speed is 1.56 times the influence of the nitrogen gas concentration $x_{N2}$ according to the fourth term of the right side. Therefore, it was considered that while there is a slight difference between the formula (13) and the formula (15), correcting the arithmetic operation by the formula (15) can provide a value substantially equal to the calculation result of the formula (13).

Accordingly, in the present embodiment, to make the calculation results of the combustion speed by the formula (13) and the formula (15) substantially equal to each other, C included in the correction term in the formula (15) was set to 1.1 times larger than A included in the correction term of the formula (13.1) (see the formula (15.1)).

For the value (referred to as "adjustment coefficient F") to be multiplied to A in the formula (15.1), a simulation was performed to compare the conventional calculation method of combustion speed (the method of obtaining a value on the basis of the (Pa) formula) and the calculation method of combustion speed using the Opt-Sonic arithmetic operation (the calculation method by the formula (15)) for natural gases with various gas compositions. As a result, a value that can reduce the error from the combustion speed calculated from the (Pa) formula, for both the natural gas with a high amount of a carbon dioxide gas and the natural gas with a high amount of a nitrogen gas, was selected as the adjustment coefficient F of the formula (15.1).

The adjustment coefficient F is 1.1 (F = 1.1) in the above-described example. However, without being limited thereto, the adjustment coefficient F is an appropriate value (for example, $0.5 \leq F \leq 1.5$) in accordance with the types of natural gases.

Thus, according to the calculation device and the calculation method of the present embodiment, it is possible to calculate the virtual basic heating value $H'_{HC}$ using the Opt-Sonic arithmetic operation and to calculate physical quantities (for example, the combustion speed) relating to the combustion of a natural gas by using the virtual basic heating value $H'_{HC}$ and the adjustment coefficient F as a substitute of the basic heating value $H_{HC}$. In this case, the adjustment coefficient F can be set and adjusted to an appropriate value (1.1 in this case). For example, the calculation device 1 of the present embodiment can hold the adjustment coefficient F as a preset value (for example, an initial value), or can receive an input of any adjustment coefficient F from the outside. In the program of the present embodiment, the adjustment coefficient F may be held as a preset value (a constant or an initial value), or the adjustment coefficient F may be one of the input parameters.

As a result, both the combustion speed $MCP(x_{N2}, x_{CO2})$ calculated by the formula (13) and the combustion speed MCP calculated by the formula (15) can produce equal values for the same type of natural gases under the same conditions.

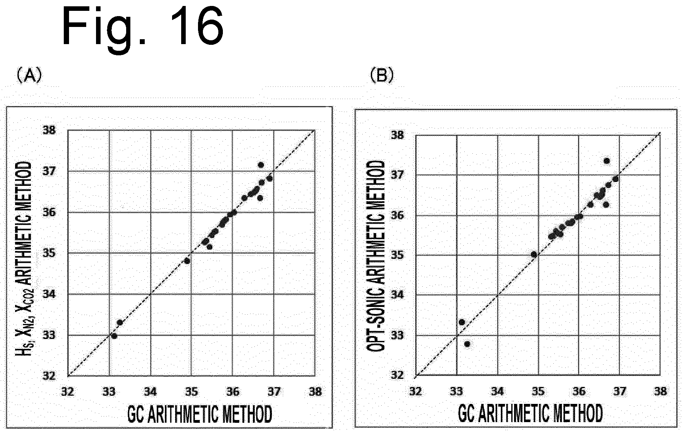

Fig. 16 shows graphs comparing the combustion speed calculated by the conventional (Pa) formula with the combustion speeds calculated by the formulas (13) and (15). In Fig 16(A), the horizontal axis represents the combustion speed MCP obtained from the (Pa) formula, and the vertical axis represents the combustion speed $MCP (x_{N2}, x_{CO2})$ obtained from the formula (13). In Fig 16(B), the horizontal axis represents the combustion speed MCP obtained from the (Pa) formula, and the vertical axis represents the combustion speed MCP obtained from the formula (15). The results of both calculations agreed with the combustion speed calculated by the conventional (Pa) formula with high accuracy.

As described in the foregoing, in the present embodiment, in calculation of the physical property values relating to a natural gas not containing, or containing a negligible amount of, hydrogen and carbon monoxide, the Opt-Sonic arithmetic operation is used to calculate the virtual basic heating value $H'_{HC}$, so that a plurality of different physical property values (for example, the compression factor, and the combustion speed), calculated on the basis of the basic heating value $H_{HC}$, can easily be calculated. For example, in general, the compression factor was conventionally calculated by the calculation methods according to ISO12213-2, ISO12213-3, or the like, and the combustion speed was calculated by the method as represented by the (Pa) formula. In these methods, the types and the number of input parameters were independently set, and therefore it was necessary to acquire (prepare) input parameters for each physical property value. Therefore, depending on the input parameters, measuring devices, analyzers, and the like were required, which caused inconvenience.

According to the present embodiment, different physical property values can be acquired by using a common calculation device and calculation method up to the stage (step) of calculating the virtual basic heating value $H'_{HC}$. The input parameters in particular are two elements of the refractive index and the density of a natural gas, and the devices for acquiring these parameters are smaller, simpler, and relatively more inexpensive than gas chromatography devices. Even in the case of calculating a plurality of different physical property values, a common device and method can be used up to the calculation of the virtual basic heating value $H'_{HC}$. Therefore, highly convenient, simple, and easy calculation can be achieved.

In the present embodiment, in calculation of the physical property values relating to a natural gas not containing, or containing a negligible amount of, hydrogen and carbon monoxide, the normalized compression factor N is calculated on the basis of three input parameters, i.e., the heating value Hs of the natural gas (a gross heating value converted to a measurement reference state temperature of 0°C, a combustion reference state of 0°C, and 101.325 kPa), the carbon dioxide gas concentration $x_{CO2}$, and the nitrogen gas concentration $x_{N2}$. Then, on the basis of the normalized compression factor N and the compression factor general formula (Z(T, P)) with the temperature T and the pressure P as parameters, the compression factor Z is calculated under the conditions of the pressure P and the temperature T to be required.

This can significantly reduce the input parameters compared to the conventional calculation methods according to ISO12213-2 and ISO12213-3. In the calculation methods according to ISO12213-2 and ISO12213-3, the pressure P and the temperature T were used as initial input data. When the compression factor was calculated under the conditions of different temperatures and pressures, all the input data required for the respective calculation methods needed to be re-calculated, and this caused a problem of inconvenience. When the temperature or pressure was not measurable, there was also a problem that numerical values relating to the compression factor could not be acquired.

According to the present embodiment, the normalized compression factor N is calculated, and the normalized compression factor N is converted to a general formula for calculating the compression factor (function Z(T, P)).

Accordingly, when the compression factor Z is calculated under the conditions of different temperatures and pressures, various compression factors Z can be calculated by changing only the input parameters, the temperature T and the pressure P in the general formula (function Z(T, P)).

In addition, calculating the normalized compression factor N using the Opt-Sonic arithmetic operation can reduce the input parameters to two elements of the refractive index and the density of a natural gas. The measurement of both the refractive index and the density can also be implemented with simpler, smaller, and less expensive devices than the conventional gas chromatography devices, so that the devices are also easy to handle.

In the present embodiment, the combustion speed relating to a natural gas, not containing, or containing a negligible amount of, hydrogen and carbon monoxide, can be calculated on the basis of three input parameters, i.e., the heating value Hs of a natural gas (a gross heating value converted to a measurement reference state temperature of 0°C, a combustion reference state of 0°C, and 101.325 kPa), the carbon dioxide gas concentration $x_{CO2}$, and the nitrogen gas concentration $x_{N2}$.

This can significantly reduce the input parameters compared to the conventional calculation method according to the (Pa) formula.

Furthermore, calculating the normalized compression factor N using the Opt-Sonic arithmetic operation can reduce the input parameters to two elements of the refractive index and the density of a natural gas. In addition, the measurement of both the refractive index and the density can be implemented with simpler, smaller, and less expensive devices than the conventional gas chromatography devices, so that the devices are also easy to handle.

The present invention is not limited to the embodiments described above but by the scope of the claims.

For example, in the embodiments described above, an example of calculating the heating value Hs using the arithmetic formula of the converted heating value (Opt-Sonic arithmetic formula) shown in the formula (7) has been described. However, the heating value Hs may be acquired by other known methods (arithmetic operations).

In the embodiment described above, the case where the necessity of measuring miscellaneous gas concentrations (the nitrogen gas concentration $x_{N2}$ and the carbon dioxide gas concentration $x_{CO2}$) is eliminated by using the Opt-Sonic arithmetic formula has been described as an example. However, at least one of the miscellaneous gases may separately be acquired (measured, for example) and then the various arithmetic operations may be performed.

For example, the target gas may also be an LNG vaporized gas or a biogas. In addition, the physical property values may include a "soot function" or an "incomplete combustion index". The miscellaneous gases are not limited to a nitrogen gas and a carbon dioxide gas. Similar arithmetic operations can be performed, and similar effects can be demonstrated with any miscellaneous gases as long as their types are limited.

Reference Signs List

| | |
|---|---|
| 1 | calculation device |
| 2 | refractive index-converted heating value acquisition unit |
| 3 | density-converted heating value acquisition unit |
| 6 | error correction value calculation unit |
| 7 | heating value calculation unit |
| 8 | miscellaneous gas total approximate-concentration calculation unit |
| 9 | virtual basic-heating-value calculation unit |
| 10 | converted heating value arithmetic unit |
| 15 | physical property value calculation unit |
| 15A | compression factor calculation unit |
| 15B | combustion speed calculation unit |
| 20 | output unit |
| 21 | refractive index measurement unit |
| 31 | sound speed measurement unit |
| 41 | gas pipeline |
| 42 | gas flow channel |
| 151 | normalized compression factor calculation unit |
| 152 | general formula conversion unit |
| z | compression factor |
| $H'_{HC}$ | virtual basic heating value |
| N | normalized compression factor |
| Z | compression factor |
| $H_{OPT}$ | refractive index-converted heating value |

$H_{SONIC}$   density-converted heating value

**Claims**

1.  A calculation device (1) configured to calculate a value relating to a combustible target gas containing miscellaneous gas components, the calculation device (1) **characterized by** comprising:

    a heating value calculation unit (7) configured to calculate a heating value, Hs, of the target gas by the following formula (A) on a basis of converted heating values, HOPT and HSONIC, obtained from a refractive index and a density of the target gas, respectively; the device being **characterized by**
    a miscellaneous gas total approximate-concentration calculation unit (8) configured to calculate a total approximate concentration, y, of the miscellaneous gas components by the following formula (B) on a basis of the converted heating values, HOPT and HSONIC; and
    a virtual basic-heating-value calculation unit (9) configured to calculate by the following formula (C), on a basis of the heating value, Hs, and the approximate concentration (y), a basic heating value, H'HC, of the target gas when the miscellaneous gas components are assumed to be removed, hereinafter referred to as a "virtual basic heating value, H'HC"

    $$H_S = H_{OPT} - (H_{OPT} - H_{SONIC})/(1-\alpha) \qquad \text{(formula A)}$$

    $$y = \psi * (H_{OPT} - H_{SONIC})/(1-\alpha) \qquad \text{(formula B)}$$

    $$H'_{HC} = H_S * 100/(100-y) \qquad \text{(formula C)}$$

    $\alpha$: correction coefficient
    $\psi$: proportional coefficient.

2.  The calculation device (1) according to claim 1, **characterized by** comprising:

    a refractive index-converted heating value acquisition unit (2) capable of acquiring the converted heating value, HOPT, obtained from the refractive index of the target gas; and
    a density-converted heating value acquisition unit (3) capable of acquiring the converted heating value, HSONIC, obtained from the density of the target gas.

3.  The calculation device (1) according to claim 1 or 2, **characterized in that**:

    the target gas is a natural gas; and
    the miscellaneous gas components mainly includes nitrogen and carbon dioxide.

4.  The calculation device (1) according to any one of claims 1 to 3, **characterized by** comprising a physical property value calculation unit (15) configured to calculate a physical property value, Z, MCP, of the target gas using the virtual basic heating value, H'HC.

5.  The calculation device (1) according to claim 4, **characterized in that** the physical property value calculation unit (15) performs error correction corresponding to the miscellaneous gas components.

6.  The calculation device (1) according to claim 4 or 5, **characterized in that** the physical property value is a compression factor, Z.

7.  The calculation device (1) according to claim 6, **characterized in that** the compression factor, Z, is calculated on a basis of a normalized compression factor, N.

8.  The calculation device (1) according to claim 4 or 5, **characterized in that** the physical property value is a combustion speed, MCP.

9.  A computer-implemented method of calculating a value relating to a combustible target gas containing a miscellaneous gas components, the calculation method **characterized by** comprising:

a step of acquiring converted heating values, HOPT and HSONIC, obtained from a refractive index and a density of the target gas, respectively (S01, S03, S301, S303, S401, S403);

a step of calculating a heating value, Hs, of the target gas on a basis of the converted heating values, HOPT and HSONIC, (S05, S305, S405);

a step of calculating a total approximate concentration of the miscellaneous gas components (hereinafter referred to as a "miscellaneous gas total approximate concentration, y" on a basis of the converted heating values, HOPT and HSONIC, (S07, S307, S407); and

a step of calculating, on a basis of the heating value, Hs, and the total approximate concentration, y, a basic heating value, H'HC, of the target gas when the miscellaneous gas components are assumed to be removed (hereinafter referred to as a "virtual basic heating value, H'HC," (S09, S309, S409).

**10.** The calculation method according to claim 9, **characterized by** comprising:

a step of acquiring the converted heating value, HOPT, obtained from the refractive index of the target gas (S01, S301, S401); and

a step of acquiring the converted heating value, HSONIC, obtained from the density of the target gas (S03, S303, S403).

**11.** The calculation method according to claim 9 or 10, **characterized in that**:

the target gas is a natural gas; and

the miscellaneous gas components mainly includes nitrogen and carbon dioxide.

**12.** The calculation method according to any one of claims 9 to 11, **characterized by** comprising a step of calculating a physical property value, Z, MCP, of the target gas using the virtual basic heating value, H'HC, (S11, S315, S411).

**13.** The calculation method according to claim 12, **characterized in that** when the physical property value, Z, MCP, is calculated, error correction corresponding to the miscellaneous gas components are performed.

**14.** The calculation method according to claim 12 or 13, **characterized in that** the physical property value, Z, MCP, is a compression factor, Z.

**15.** The calculation method according to claim 14, **characterized in that** the compression factor, Z, is calculated on a basis of a normalized compression factor, N.

**16.** The calculation method according to claim 12 or 13, **characterized in that** the physical property value, Z, MCP, is a combustion speed, MCP.

**17.** A program for causing a computer to execute the calculation method according to any one of claims 9 to 16.

## Patentansprüche

**1.** Berechnungsvorrichtung (1), die zum Berechnen eines Wertes in Bezug auf ein brennbares Zielgas, das verschiedene Gaskomponenten enthält, konfiguriert ist, wobei die Berechnungsvorrichtung (1) **dadurch gekennzeichnet ist, dass** sie Folgendes umfasst:

eine Heizwertberechnungseinheit (7), die konfiguriert ist, um einen Heizwert, Hs, des Zielgases anhand der folgenden Formel (A) auf einer Grundlage von umgerechneten Heizwerten, HOPT und HSONIC, zu berechnen, die aus einem Brechungsindex bzw. einer Dichte des Zielgases gewonnen werden; wobei das Gerät **gekennzeichnet ist durch**:

eine Berechnungseinheit für die ungefähre Gesamtkonzentration verschiedener Gase (8), die konfiguriert ist, um eine ungefähre Gesamtkonzentration y der verschiedenen Gaskomponenten anhand der folgenden Formel (B) auf einer Grundlage der umgerechneten Heizwerte, HOPT und HSONIC, zu berechnen; und

eine virtuelle Grundheizwert-Berechnungseinheit (9), die konfiguriert ist, um anhand der folgenden Formel (C) auf einer Grundlage des Heizwerts Hs und der ungefähren Konzentration (y) einen Grundheizwert H'HC des Zielgases zu berechnen, wenn davon ausgegangen wird, dass die verschiedenen Gaskomponenten entfernt sind, im Folgenden als "virtueller Grundheizwert H'HC" bezeichnet

$$H_S = H_{OPT} - (H_{OPT} - H_{SONIC})/(1-\alpha) \qquad \text{(Formel A)}$$

$$y = \psi * (H_{OPT} - H_{SONIC})/(1-\alpha) \qquad \text{(Formel B)}$$

$$H'_{HC} = H_S * 100/(100-y) \qquad \text{(Formel C)}$$

$\alpha$: Korrekturkoeffizient
$\psi$: Proportionalitätskoeffizient.

2. Berechnungsvorrichtung (1) gemäß Anspruch 1, **dadurch gekennzeichnet ist, dass** sie Folgendes umfasst:

eine Einheit zur Erfassung des in den Brechungsindex umgerechneten Heizwerts (2), die in der Lage ist, den aus dem Brechungsindex des Zielgases erhaltenen umgerechneten Heizwert HOPT zu erfassen; und
eine Einheit zur Erfassung des in die Dichte umgerechneten Heizwerts (3), die in der Lage ist, den aus der Dichte des Zielgases erhaltenen umgerechneten Heizwert HSONIC zu erfassen.

3. Berechnungsvorrichtung (1) gemäß Anspruch 1 oder 2, **dadurch gekennzeichnet, dass**:

das Zielgas ein Erdgas ist; und
die verschiedenen Gaskomponenten hauptsächlich Stickstoff und Kohlendioxid enthalten.

4. Berechnungsvorrichtung (1) gemäß einem der Ansprüche 1 bis 3, **dadurch gekennzeichnet, dass** sie eine Berechnungseinheit für physikalische Eigenschaftswerte (15) umfasst, die konfiguriert ist, um einen physikalischen Eigenschaftswert, Z, MCP, des Zielgases unter Verwendung des virtuellen Grundheizwerts H'HC zu berechnen.

5. Berechnungsvorrichtung (1) gemäß Anspruch 4, **dadurch gekennzeichnet, dass** die Berechnungseinheit für physikalische Eigenschaftswerte (15) eine Fehlerkorrektur entsprechend den verschiedenen Gaskomponenten durchführt.

6. Berechnungsvorrichtung (1) gemäß Anspruch 4 oder 5, **dadurch gekennzeichnet, dass** der physikalische Eigenschaftswert ein Kompressionsfaktor Z ist.

7. Berechnungsvorrichtung (1) gemäß Anspruch 6, **dadurch gekennzeichnet, dass** der Kompressionsfaktor Z auf einer Grundlage eines normalisierten Kompressionsfaktors N berechnet wird.

8. Berechnungsvorrichtung (1) gemäß Anspruch 4 oder 5, **dadurch gekennzeichnet, dass** der physikalische Eigenschaftswert eine Verbrennungsgeschwindigkeit MCP ist.

9. Computerimplementiertes Verfahren zum Berechnen eines Wertes, der sich auf ein brennbares Zielgas bezieht, das verschiedene Gaskomponenten enthält, wobei das Berechnungsverfahren **dadurch gekennzeichnet ist, dass** es Folgendes umfasst:

einen Schritt zum Erfassen umgerechneter Heizwerte, HOPT und HSONIC, die aus einem Brechungsindex bzw. einer Dichte des Zielgases gewonnen werden (S01, S03, S301, S303, S401, S403);
einen Schritt zum Berechnen eines Heizwerts, Hs, des Zielgases auf einer Grundlage der umgewandelten Heizwerte HOPT und HSONIC (S05, S305, S405);
einen Schritt zum Berechnen einer ungefähren Gesamtkonzentration der verschiedenen Gaskomponenten (im Folgenden als "ungefähre Gesamtkonzentration der verschiedenen Gase, y" bezeichnet) auf einer Grundlage der umgewandelten Heizwerte HOPT und HSONIC (S07, S307, S407); und
einen Schritt zum Berechnen eines Grundheizwerts, H'HC, des Zielgases auf einer Grundlage des Heizwerts Hs und der ungefähren Gesamtkonzentration y, wenn angenommen wird, dass die verschiedenen Gaskomponenten entfernt wurden (im Folgenden als "virtueller Grundheizwert H'HC" bezeichnet) (S09, S309, S409).

10. Berechnungsverfahren gemäß Anspruch 9, **dadurch gekennzeichnet ist, dass** es Folgendes umfasst:

einen Schritt zum Erfassen des umgerechneten Heizwerts HOPT, der aus dem Brechungsindex des Zielgases gewonnen wird (S01, S301, S401); und
einen Schritt zum Erfassen des umgerechneten Heizwerts HSONIC, der aus der Dichte des Zielgases gewonnen wird (S03, S303, S403).

11. Berechnungsverfahren gemäß Anspruch 9 oder 10, **dadurch gekennzeichnet, dass**:

das Zielgas ein Erdgas ist; und
die verschiedenen Gaskomponenten hauptsächlich Stickstoff und Kohlendioxid enthalten.

12. Berechnungsverfahren gemäß einem der Ansprüche 9 bis 11, **dadurch gekennzeichnet, dass** es einen Schritt zum Berechnen eines physikalischen Eigenschaftswerts, Z, MCP, des Zielgases unter Verwendung des virtuellen Grundheizwerts H'HC (S11, S315, S411) umfasst.

13. Berechnungsverfahren gemäß Anspruch 12, **dadurch gekennzeichnet, dass** bei der Berechnung des physikalischen Eigenschaftswerts Z, MCP eine Fehlerkorrektur entsprechend den verschiedenen Gaskomponenten durchgeführt wird.

14. Berechnungsverfahren gemäß Anspruch 12 oder 13, **dadurch gekennzeichnet, dass** der physikalische Eigenschaftswert Z, MCP ein Kompressionsfaktor Z ist.

15. Berechnungsverfahren gemäß Anspruch 14, **dadurch gekennzeichnet, dass** der Kompressionsfaktor Z auf einer Grundlage eines normalisierten Kompressionsfaktors N berechnet wird.

16. Berechnungsverfahren gemäß Anspruch 12 oder 13, **dadurch gekennzeichnet, dass** der physikalische Eigenschaftswert Z, MCP eine Verbrennungsgeschwindigkeit MCP ist.

17. Programm, das einen Computer veranlasst, das Berechnungsverfahren gemäß einem der Ansprüche 9 bis 16 auszuführen.

## Revendications

1. Dispositif de calcul (1) configuré pour calculer une valeur relative à un gaz cible combustible contenant des composants gazeux divers, le dispositif de calcul (1) étant **caractérisé en ce qu'**il comprend :

une unité de calcul de pouvoir calorifique (7) configurée pour calculer un pouvoir calorifique, Hs, du gaz cible à l'aide de la formule (A) suivante sur une base des pouvoirs calorifiques convertis, HOPT et HSONIC, obtenus respectivement à partir de l'indice de réfraction et de la densité du gaz cible ; le dispositif étant **caractérisé par** une unité de calcul de la concentration approximative totale des gaz divers (8) configurée pour calculer une concentration approximative totale, y, des composants gazeux divers à l'aide de la formule suivante (B) sur une base des pouvoirs calorifiques convertis, HOPT et HSONIC ; et
une unité de calcul du pouvoir calorifique de base virtuelle (9) configurée pour calculer, à l'aide de la formule (C) suivante, sur une base du pouvoir calorifique, Hs, et de la concentration approximative (y), un pouvoir calorifique de base, H'HC, du gaz cible lorsque les composants gazeux divers sont supposés être éliminés, ci-après dénommé « pouvoir calorifique de base virtuelle, H'HC »

$$H_S = H_{OPT} - (H_{OPT} - H_{SONIC})/(1-\alpha) \qquad \text{(formule A)}$$

$$y = \psi * (H_{OPT} - H_{SONIC})/(1-\alpha) \qquad \text{(formule B)}$$

$$H'_{HC} = H_S * 100/(100-y) \qquad \text{(formule C)}$$

$\alpha$ : coefficient de correction

$\psi$ : coefficient proportionnel.

2.  Dispositif de calcul (1) selon la revendication 1, **caractérisé en ce qu'**il comprend :

    une unité d'acquisition du pouvoir calorifique converti en indice de réfraction (2) capable d'acquérir le pouvoir calorifique converti, HOPT, obtenue à partir de l'indice de réfraction du gaz cible ; et
    une unité d'acquisition du pouvoir calorifique converti en densité (3) capable d'acquérir le pouvoir calorifique converti, HSONIC, obtenue à partir de la densité du gaz cible.

3.  Dispositif de calcul (1) selon la revendication 1 ou 2, **caractérisé en ce que** :

    le gaz cible est un gaz naturel ; et
    les composants gazeux divers comportent principalement de l'azote et du dioxyde de carbone.

4.  Dispositif de calcul (1) selon l'une quelconque des revendications 1 à 3, **caractérisé en ce qu'**il comprend une unité de calcul de valeur de propriété physique (15) configurée pour calculer une valeur de propriété physique, Z, MCP, du gaz cible en utilisant le pouvoir calorifique de base virtuel, H'HC.

5.  Dispositif de calcul (1) selon la revendication 4, **caractérisé en ce que** l'unité de calcul de la valeur de propriété physique (15) effectue une correction d'erreur correspondant aux composants gazeux divers.

6.  Dispositif de calcul (1) selon la revendication 4 ou 5, **caractérisé en ce que** la valeur de propriété physique est un facteur de compression, Z.

7.  Dispositif de calcul (1) selon la revendication 6, **caractérisé en ce que** le facteur de compression, Z, est calculé sur une base d'un facteur de compression normalisé, N.

8.  Dispositif de calcul (1) selon la revendication 4 ou 5, **caractérisé en ce que** la valeur de propriété physique est une vitesse de combustion, MCP.

9.  Procédé mis en œuvre par ordinateur pour calculer une valeur relative à un gaz cible combustible contenant des composants gazeux divers, le procédé de calcul étant **caractérisé en ce qu'**il comprend :

    une étape consistant à acquérir des pouvoirs calorifiques convertis, HOPT et HSONIC, obtenues respectivement à partir d'un indice de réfraction et d'une densité du gaz cible (S01, S03, S301, S303, S401, S403) ;
    une étape consistant à calculer un pouvoir calorifique, Hs, du gaz cible sur une base des pouvoirs calorifiques convertis, HOPT et HSONIC, (S05, S305, S405) ;
    une étape consistant à calculer une concentration approximative totale des composants gazeux divers (ci-après dénommée « concentration approximative totale des gaz divers, y ») sur une base des pouvoirs calorifiques convertis, HOPT et HSONIC (S07, S307, S407) ; et
    une étape consistant à calculer, sur une base du pouvoir calorifique, Hs, et de la concentration approximative totale, y, un pouvoir calorifique de base, H'HC, du gaz cible lorsque les composants gazeux divers sont supposés être éliminés (ci-après dénommée « pouvoir calorifique de base virtuelle, H'HC ») (S09, S309, S409).

10. Procédé de calcul selon la revendication 9, **caractérisé en ce qu'**il comprend :

    une étape consistant à acquérir le pouvoir calorifique converti, HOPT, obtenu à partir de l'indice de réfraction du gaz cible (S01, S301, S401) ; et
    une étape consistant à acquérir le pouvoir calorifique converti, HSONIC, obtenu à partir de la densité du gaz cible (S03, S303, S403).

11. Procédé de calcul selon la revendication 9 ou 10, **caractérisé en ce que** :

    le gaz cible est un gaz naturel ; et
    les composants gazeux divers comportent principalement de l'azote et du dioxyde de carbone.

12. Procédé de calcul selon l'une quelconque des revendications 9 à 11, **caractérisé en ce qu'**il comprend une étape consistant à calculer une valeur de propriété physique, Z, MCP, du gaz cible en utilisant le pouvoir calorifique de base virtuelle, H'HC, (S11, S315, S411).

13. Procédé de calcul selon la revendication 12, **caractérisé en ce que**, lorsque la valeur de propriété physique, Z, MCP, est calculée, une correction d'erreur correspondant aux composants gazeux divers est effectuée.

14. Procédé de calcul selon la revendication 12 ou 13, **caractérisé en ce que** la valeur de propriété physique, Z, MCP, est un facteur de compression, Z.

15. Procédé de calcul selon la revendication 14, **caractérisé en ce que** le facteur de compression, Z, est calculé sur une base d'un facteur de compression normalisé, N.

16. Procédé de calcul selon la revendication 12 ou 13, **caractérisé en ce que** la valeur de propriété physique, Z, MCP, est une vitesse de combustion, MCP.

17. Programme destiné à faire exécuter par un ordinateur le procédé de calcul selon l'une quelconque des revendications 9 à 16.

# Fig. 1

REFRACTIVE INDEX-CONVERTED HEATING VALUE ACQUISITION UNIT

REFRACTIVE INDEX MEASUREMENT UNIT

REFRACTIVE INDEX-TO-HEATING VALUE CONVERSION PROCESSING UNIT

REFRACTIVE INDEX-CONVERTED HEATING VALUE $H_{OPT}$

DENSITY-CONVERTED HEATING VALUE ACQUISITION UNIT

SOUND SPEED MEASUREMENT UNIT

DENSITY-TO-HEATING VALUE CONVERSION PROCESSING UNIT

DENSITY-CONVERTED HEATING VALUE $H_{SONIC}$

$$\frac{H_{OPT} - H_{SONIC}}{1 - \alpha}$$

ERROR CORRECTION VALUE CALCULATION UNIT

HEATING VALUE CALCULATION UNIT

HEATING VALUE Hs

$$Hs = H_{OPT} - \frac{H_{OPT} - H_{SONIC}}{1 - \alpha}$$

CONVERTED HEATING VALUE ARITHMETIC UNIT

$$(x_{N2} + 1.56 \times x_{CO2}) = y$$

MISCELLANEOUS GAS TOTAL APPROXIMATE-CONCENTRATION CALCULATION UNIT

MISCELLANEOUS GAS TOTAL APPROXIMATE CONCENTRATION y

VIRTUAL BASIC-HEATING-VALUE CALCULATION UNIT

VIRTUAL BASIC HEATING VALUE $H'_{HC}$

$$H'_{HC} = Hs \times \frac{100}{100 - y} = Hs \times \frac{100}{100 - (x_{N2} + 1.56 \times x_{CO2})}$$

OUTPUT UNIT

28

# Fig. 2

(A)

**CONVERTED HEATING VALUE ARITHMETIC UNIT** — 10, 1

**HEATING VALUE CALCULATION UNIT** — 7

HEATING VALUE Hs

**MISCELLANEOUS GAS TOTAL APPROXIMATE-CONCENTRATION CALCULATION UNIT** — 8

MISCELLANEOUS GAS TOTAL APPROXIMATE CONCENTRATION y

**VIRTUAL BASIC-HEATING-VALUE CALCULATION UNIT** — 9

VIRTUAL BASIC HEATING VALUE $H'_{HC}$

**PHYSICAL PROPERTY VALUE CALCULATION UNIT** — 15

**OUTPUT UNIT** — 20

(B)

**PHYSICAL PROPERTY VALUE CALCULATION UNIT** — 15A

**COMPRESSION FACTOR CALCULATION UNIT**

**NORMALIZED COMPRESSION FACTOR CALCULATION UNIT** — 151

NORMALIZED COMPRESSION FACTOR N

$$N_{39.933MJ}^{46.547MJ} = N'_{HC} + k'x_{N2} + 1.56 \times k'x_{CO2}$$

**GENERAL FORMULA CONVERSION UNIT** — 152

$Z(T, P)$

PRESSURE P
TEMPERATURE T

COMPRESSION FACTOR Z

(C)

**PHYSICAL PROPERTY VALUE CALCULATION UNIT**

**COMBUSTION SPEED CALCULATION UNIT** — 15B

$$MCP = -0.004637 \times H'^2_{HC} + 0.5961 \times H'_{HC} + 19.5965 + C \times (x_{N2} + 1.56 \times x_{CO2})$$

COMBUSTION SPEED MCP

# Fig. 3

(A)

```
┌─────────────────────────────┐        ┌─────────────────────────────┐
│      REFRACTIVE INDEX        │        │          DENSITY            │
└─────────────────────────────┘        └─────────────────────────────┘
```

┌─────────────────────────────────────────────────────────────────────────┐
│  ┌──────────────────────────────┐ ~S01  ┌──────────────────────────────┐ ~S03
│  │ REFRACTIVE INDEX-CONVERTED   │        │  DENSITY-CONVERTED           │
│  │ HEATING VALUE $H_{OPT}$      │        │  HEATING VALUE $H_{SONIC}$   │
│  └──────────────────────────────┘        └──────────────────────────────┘
│                                                                    ~S05
│              ┌──────────────────────────────────────┐
│              │  CALCULATE HEATING VALUE Hs          │
│              │  OF TARGET GAS                       │
│              └──────────────────────────────────────┘
│   HEATING VALUE Hs                    OPT-SONIC ARITHMETIC OPERATION
└─────────────────────────────────────────────────────────────────────────┘

              ┌──────────────────────────────────────┐ ~S07
              │  CALCULATE MISCELLANEOUS GAS          │
              │  TOTAL APPROXIMATE CONCENTRATION y    │
              └──────────────────────────────────────┘
   MISCELLANEOUS GAS TOTAL
   APPROXIMATE CONCENTRATION y
              ┌──────────────────────────────────────┐ ~S09
              │  CALCULATE VIRTUAL                    │
              │  BASIC HEATING VALUE $H'_{HC}$        │
              └──────────────────────────────────────┘
   VIRTUAL BASIC
   HEATING VALUE $H'_{HC}$
              ┌──────────────────────────────────────┐ ~S11
              │  CALCULATE PHYSICAL                   │
              │  PROPERTY VALUE                       │
              └──────────────────────────────────────┘

(B)                                          (C)

┌──────────────────────────────┐            ┌──────────────────────────────┐
│  CALCULATE PHYSICAL          │            │  CALCULATE PHYSICAL          │
│  PROPERTY VALUE              │            │  PROPERTY VALUE              │
└──────────────────────────────┘            └──────────────────────────────┘

S111    ┌──────────────────────────────┐    ┌──────────────────────────────┐
        │ CALCULATE NORMALIZED         │    │ CALCULATE COMBUSTION SPEED   │
        │ COMPRESSION FACTOR N ON THE  │    │ MCP ON THE BASIS OF          │
        │ BASIS OF MISCELLANEOUS GAS   │    │ MISCELLANEOUS GAS            │
        │ TOTAL APPROXIMATE            │    │ TOTAL APPROXIMATE            │
        │ CONCENTRATION y AND          │    │ CONCENTRATION y AND          │
        │ VIRTUAL BASIC HEATING        │    │ VIRTUAL BASIC HEATING        │
        │ VALUE $H'_{HC}$,             │    │ VALUE $H'_{HC}$,             │
        │ AND CORRECT ERROR            │    │ AND CORRECT ERROR            │
        └──────────────────────────────┘    └──────────────────────────────┘

S112    ┌──────────────────────────────┐
        │ CONNVERT NORMALIZED          │
        │ COMPRESSION FACTOR N INTO    │
        │ GENERAL FORMULA              │
        │ (FUNCTION Z(T, P))           │
        └──────────────────────────────┘

   ┌ ─ ─ ─ ─ ─ ─ ─ ─ ┐
   │ PRESSURE P       │────────▶
   │ TEMPERATURE T    │
   └ ─ ─ ─ ─ ─ ─ ─ ─ ┘

S113    ┌──────────────────────────────┐
        │ CALCULATE COMPRESSION FACTOR Z│
        └──────────────────────────────┘

# Fig. 4

(A)

| HEATING VALUE Hs | CARBON DIOXIDE GAS CONCENTRATION $X_{CO2}$ | NITROGEN GAS CONCENTRATION $X_{N2}$ |
|---|---|---|

$$H_{HC} = H_S \times \frac{100}{100 - (x_{N2} + x_{CO2})}$$

CALCULATE NORMALIZED COMPRESSION FACTOR N, FORMULA (5) — S201

$$N_{39.933MJ}^{46.547MJ}(x_{N2}, x_{CO2}) = N_{HC} + k_{N2}x_{N2} + k_{CO2}x_{CO2}$$

CONVERT TO GENERAL FORMULA Z(T,P) — S203

CALCULATE COMPRESSION FACTOR Z — S205

(B)

| REFRACTIVE INDEX | DENSITY |
|---|---|

REFRACTIVE INDEX-CONVERTED HEATING VALUE — S301

DENSITY-CONVERTED HEATING VALUE — S303

$$Hs = H_{OPT} - \frac{H_{OPT} - H_{SONIC}}{1 - \alpha}$$

CALCULATE HEATING VALUE Hs OF TARGET GAS — S305

OPT-SONIC ARITHMETIC

CALCULATE MISCELLANEOUS GAS TOTAL APPROXIMATE CONCENTRATION y — S307

CALCULATE VIRTUAL BASIC HEATING VALUE H'$_{HC}$ — S309

CALCULATE NORMALIZED COMPRESSION FACTOR N, FORMULA (10) — S311

$$N_{39.933MJ}^{46.547MJ} = N'_{HC} + k'x_{N2} + 1.56 \times k'x_{CO2}$$

CONVERT TO GENERAL FORMULA — S313

CALCULATE COMPRESSION FACTOR Z — S315

# Fig. 5

(A)

| HEATING VALUE Hs | CARBON DIOXIDE GAS CONCENTRATION $X_{CO2}$ | NITROGEN GAS CONCENTRATION $X_{N2}$ |
|---|---|---|

CALCULATE COMBUSTION SPEED, FORMULA (13)

$$H_{HC} = H_S \times \frac{100}{100 - (x_{N2} + x_{CO2})}$$

$$MCP = -0.004637 \times H_{HC}^2 + 0.5961 \times H_{HC} + 19.5965 + A \times x_{N2} + B \times x_{CO2}$$

(B)

| REFRACTIVE INDEX | DENSITY |
|---|---|

REFRACTIVE INDEX-CONVERTED HEATING VALUE   S401

DENSITY-CONVERTED HEATING VALUE   S403

CALCULATE HEATING VALUE Hs OF TARGET GAS   S405

$$Hs = H_{OPT} - \frac{H_{OPT} - H_{SONIC}}{1 - \alpha}$$

OPT-SONIC ARITHMETIC OPERATION

CALCULATE MISCELLANEOUS GAS TOTAL APPROXIMATE CONCENTRATION y   S407

CALCULATE VIRTUAL BASIC HEATING VALUE $H'_{HC}$   S409

CALCULATE COMBUSTION SPEED, FORMULA (15)   S411

$$MCP = -0.004637 \times H'^2_{HC} + 0.5961 \times H'_{HC} + 19.5965 + C \times (x_{N2} + 1.56 \times x_{CO2})$$

# Fig. 6

Fig. 7

# Fig. 8

# Fig. 9

PRESSURE CONDITION: 0.101325 MPa

# Fig. 10

# Fig. 11

PRESSURE CONDITION: 5 MPa

# Fig. 12

PRESSURE CONDITION: 7 MPa

# Fig. 13

# Fig. 14

# Fig. 15

(A)

(B)

EP 4 328 585 B1

# Fig. 16

(A)

(B)

**EP 4 328 585 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6402387 B
- WO 2020148927 A1
- JP 2021004891 A
- WO 2016104270 A1